(19) Europäisches Patentamt / European Patent Office / Office européen des brevets

(11) **EP 4 109 098 B1**

(12) **EUROPEAN PATENT SPECIFICATION**

(45) Date of publication and mention
of the grant of the patent:
**23.07.2025   Bulletin 2025/30**

(51) International Patent Classification (IPC):
*G01N 33/53* *(2006.01)*

(52) Cooperative Patent Classification (CPC):
**G01N 33/5306**

(21) Application number: **22165153.2**

(22) Date of filing: **29.03.2022**

(54) **DETECTION METHOD FOR DETECTING OCCURRENCE OF NONSPECIFIC REACTION, ANALYSIS METHOD, ANALYZER, AND DETECTION PROGRAM FOR DETECTING OCCURRENCE OF NONSPECIFIC REACTION**

DETEKTIONSVERFAHREN ZUR DETEKTION DES AUFTRETENS EINER UNSPEZIFISCHEN REAKTION, ANALYSEVERFAHREN, ANALYSATOR UND DETEKTIONSPROGRAMM ZUR DETEKTION DES AUFTRETENS EINER UNSPEZIFISCHEN REAKTION

PROCÉDÉ DE DÉTECTION DE L'APPARITION D'UNE RÉACTION NON SPÉCIFIQUE, PROCÉDÉ D'ANALYSE, DISPOSITIF D'ANALYSE ET PROGRAMME DE DÉTECTION DE L'APPARITION D'UNE RÉACTION NON SPÉCIFIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **23.06.2021   JP 2021104323**

(43) Date of publication of application:
**28.12.2022   Bulletin 2022/52**

(73) Proprietor: **SYSMEX CORPORATION
Kobe-shi
Hyogo 651-0073 (JP)**

(72) Inventors:
• **TABUCHI, Yuka**
  **Kobe-shi, Hyogo, 651-0073 (JP)**
• **KIMURA, Konobu**
  **Kobe-shi, Hyogo, 651-0073 (JP)**
• **NISHI, Keisuke**
  **Kobe-shi, Hyogo, 651-0073 (JP)**
• **KUMANO, Osamu**
  **Kobe-shi, Hyogo, 651-0073 (JP)**
• **SUZUKI, Takeshi**
  **Kobe-shi, Hyogo, 651-0073 (JP)**

(74) Representative: **Hoffmann Eitle
Patent- und Rechtsanwälte PartmbB
Arabellastraße 30
81925 München (DE)**

(56) References cited:
• FRUTIGER ANDREAS ET AL: "Nonspecific Binding-Fundamental Concepts and Consequences for Biosensing Applications", CHEMICAL REVIEWS, vol. 121, no. 13, 9 June 2021 (2021-06-09), US, pages 8095 - 8160, XP055959632, ISSN: 0009-2665, DOI: 10.1021/acs.chemrev.1c00044
• BALLARD ZACHARY S. ET AL: "Deep learning-enabled point-of-care sensing using multiplexed paper-based sensors", NPJ DIGITAL MEDICINE, vol. 3, no. 1, 7 May 2020 (2020-05-07), XP055959616, Retrieved from the Internet <URL:http://www.nature.com/articles/s41746-020-0274-y> DOI: 10.1038/s41746-020-0274-y
• BANAEI NARIMAN ET AL: "Machine learning algorithms enhance the specificity of cancer biomarker detection using SERS-based immunoassays in microfluidic chips", RSC ADVANCES, vol. 9, no. 4, 15 January 2019 (2019-01-15), pages 1859 - 1868, XP055959617, Retrieved from the Internet <URL:https://pubs.rsc.org/en/content/articlepdf/2019/ra/c8ra08930b> DOI: 10.1039/C8RA08930B

• **YAN WENQIANG ET AL: "Machine Learning Approach to Enhance the Performance of MNP-Labeled Lateral Flow Immunoassay", NANO-MICRO LETTERS, vol. 11, no. 1, 1 December 2019 (2019-12-01), XP055959624, ISSN: 2311-6706, Retrieved from the Internet <URL:https://link.springer.com/content/pdf/10.1007/s40820-019-0239-3.pdf> DOI: 10.1007/s40820-019-0239-3**

**Description**

FIELD OF THE INVENTION

The present invention relates to a detection method for detecting occurrence of nonspecific reaction, an analysis method, an analyzer, and a detection program for detecting occurrence of nonspecific reaction as defined in the appended claims.

BACKGROUND OF THE INVENTION

As a method for measuring an antigen or an antibody, immunoassay using antigen-antibody reaction is generally used for testing a measurement sample which is derived from an organism and contains blood, urine, or the like. It is generally known that, in the test using the immunoassay, nonspecific reaction progresses due to rheumatoid factor, complement component C1q, or the like which is mixed in a biological sample such as blood or urine, and the test result may indicate false-positive. EP Patent Application Publication No. 0566205 discloses a method for reducing influence caused by such nonspecific reaction.

According to EP Patent Application Publication No. 0566205, an antibody, in which a constant region (Fc portion) that has antibody amino acid sequences indicating high similarity and that may cause such nonspecific reaction is blocked by a blocking agent, is used to reduce nonspecific reaction.

However, even when the method described in EP Patent Application Publication No. 0566205 is used, it is difficult to completely inhibit occurrence of nonspecific reaction in the immunoassay. Occurrence of nonspecific reaction makes a test result false-positive. Therefore, occurrence of nonspecific reaction needs to be detected to prevent report of false-positive test results.

Frutiger, Andreas, et al. is a review on fundamental concepts and consequences for biosensing application discussing existing practical approaches to tackle the nonspecific binding challenge *in vitro* for biosensing platforms and how to address and harness nonspecific interactions for *in vivo* systems. (Frutiger, Andreas, et al. "Nonspecific binding-fundamental concepts and consequences for biosensing applications." Chemical reviews 121.13 (2021): 8095-8160).

An object of the present invention is to provide a detection method for detecting occurrence of nonspecific reaction, an analysis method, an analyzer, and a detection program for detecting occurrence of nonspecific reaction, which are capable of detecting occurrence of nonspecific reaction in an analysis system using antigen-antibody reaction.

SUMMARY OF THE INVENTION

The invention relates to the following embodiments:

1. A computer-implemented detection method for detecting occurrence of nonspecific reaction in analysis for an antigen or an antibody contained in a biological sample with use of a measurement reagent containing an antibody or an antigen that causes antigen-antibody reaction with the antigen or the antibody in the biological sample, the detection method comprising:

generating a data group about progress of antigen-antibody reaction between the antigen or the antibody contained in the biological sample and the antibody or the antigen contained in the measurement reagent;
inputting the data group to a deep learning algorithm; and
generating information about occurrence of nonspecific reaction, based on a result outputted by the deep learning algorithm, wherein
the generating of the data group comprises receiving detection information indicating an intensity of detected light that is detected through a measurement sample to which light is applied for a predetermined time period, and generating the data group from the detection information having been received, wherein each piece of data included in the data group expresses an intensity of the detected light that is detected at each measurement time point in the predetermined time period.

2. The detection method of item 1, wherein each piece of data included in the data group indicates an intensity of light transmitted through the measurement sample, an intensity of light absorbed by the measurement sample, or an intensity of light scattered by the measurement sample.

3. The detection method of item 1 or 2, wherein

the detection information indicates an intensity of the detected light that is detected through the measurement sample to which a plurality of kinds of lights having different wavelengths are sequentially applied,
the generating of the data group comprises generating a plurality of the data groups corresponding to the lights, respectively, based on the detection information, and
the inputting of the data group to the deep learning algorithm comprises inputting a plurality of the data groups to the deep learning algorithm.

4. An analysis method for analyzing an antigen or an antibody contained in a biological sample by using a measurement reagent containing an antibody or an antigen that causes antigen-antibody reaction with the antigen or the antibody in the biological sample, the analysis method comprising:

preparing a measurement sample containing the biological sample and the measurement reagent;
analyzing the antigen or the antibody contained in the biological sample; and
performing the detection method of item 1 for detecting occurrence of the nonspecific reaction.

5. The analysis method of item 4, further comprising

applying light to the measurement sample for a predetermined time period and outputting detection information indicating an intensity of detected light that is detected through the measurement sample, wherein
the analyzing of the antigen or the antibody contained in the biological sample comprises analyzing the antigen or the antibody based on the detection information.

6. The analysis method of item 5, wherein

the generating of the data group comprises
generating the data group from the detection information, and
the analyzing of the antigen or the antibody contained in the biological sample comprises
analyzing the antigen or the antibody based on the data group generated from the detection information.

7. The analysis method of any one of items 4 to 6, wherein, when a result of the analysis of the antigen or the antibody contained in the biological sample satisfies a first condition, the detection method for detecting occurrence of the nonspecific reaction is performed.

8. The analysis method of any one of items 4 to 7, wherein, when a result outputted from the deep learning algorithm satisfies a second condition, information about occurrence of the nonspecific reaction is outputted.

9. The analysis method of item 8, wherein
the outputting of the information about occurrence of the nonspecific reaction further comprises
outputting an analysis result of a previous biological sample of a subject from whom the biological sample has been collected, and/or information about clinical information obtained by diagnosing the subject.

10. The analysis method of item 8 or 9, further comprising outputting a message for suggesting re-analysis of the biological sample when a result outputted from the deep learning algorithm satisfies the second condition.

11. The analysis method of item 10, further comprising

receiving an execution instruction for executing re-analysis of the biological sample when a result outputted by the deep learning algorithm satisfies the second condition, and
executing the re-analysis of the biological sample when the execution instruction is received.

12. The analysis method of item 11, wherein the receiving of the execution instruction comprises receiving an execution instruction for analyzing an antigen or an antibody of a kind different from a kind of the antigen or the antibody contained in the biological sample.

13. The analysis method of item 11 or 12, wherein the receiving of the execution instruction comprises receiving an execution instruction for analyzing an antigen or an antibody of a same kind as a kind of the antigen or the antibody contained in the biological sample by diluting the biological sample at a dilution rate higher than a dilution rate for the measurement sample.

14. An analyzer for analyzing an antigen or an antibody contained in a biological sample by using a measurement reagent containing an antibody or an antigen that causes antigen-antibody reaction with the antigen or the antibody in the biological sample, the analyzer comprising:

a sample preparation unit configured to prepare a measurement sample containing the biological sample and the measurement reagent;
a light applying unit configured to apply light to the measurement sample;
a detector configured to detect the light applied by the light applying unit, through the measurement sample, and output detection information corresponding to the detected light; and
a controller configured to

analyze the antigen or the antibody contained in the biological sample; and
detect occurrence of nonspecific reaction in the measurement sample, wherein the detecting of occurrence of the nonspecific reaction comprises the detection method according to any one of items 1-3,
generating a data group about progress of antigen-antibody reaction between the antigen or the antibody contained in the biological sample and the antibody or the antigen contained in the measurement reagent based on the detection information,
inputting the data group to a deep learning algorithm, and
generating information about occurrence of nonspecific reaction based on a result outputted from the deep learning algorithm.

15. A detection program for detecting occurrence of nonspecific reaction, the detection program causing a computer to execute, when the computer is caused to execute the program the detection method according to any one of items 1-3

The present invention is directed to a detection program for detecting occurrence of nonspecific reaction. The detection program causes a computer to execute, when the computer is caused to execute the program, generating a data group about progress of antigen-antibody reaction between an antigen or an antibody contained in a biological sample and an antibody or an antigen contained in a measurement reagent, inputting the data group to a deep learning algorithm, and generating information about occurrence of nonspecific reaction based on a result outputted from the deep learning algorithm.

The detection method for detecting occurrence of nonspecific reaction, the analysis method, the analyzer, and the detection program for detecting occurrence of nonspecific reaction can detect occurrence of nonspecific reaction in an analysis system using antigen-antibody reaction.

Occurrence of nonspecific reaction can be detected in an analysis system using antigen-antibody reaction.

BRIEF DESCRIPTION OF THE DRAWINGS

FIG. 1 illustrates an example of an outer appearance of an analyzer 1;
FIG. 2 illustrates an example of a hardware configuration of the analyzer 1;
FIG. 3 illustrates an example of a configuration of a storage unit 202;
FIG. 4 illustrates an example of a structure of a light applying unit 10;
FIG. 5A illustrates an example of a structure of a detector 230 that does not hold a container 15;
FIG. 5B illustrates an example of a structure of the detector 230 that holds the container 15;
FIG. 6 shows a flow of an analysis process performed by a controller 70 of the analyzer 1;
FIG. 7 shows a flow of a measurement process executed by the controller 70 according to a measurement program 202a;
FIG. 8 shows a flow of a test substance analysis/nonspecific reaction detection process executed by the controller 70 according to a test substance analysis/nonspecific reaction detection program 202b;
FIG. 9A illustrates an example of data groups about progress of antigen-antibody reaction;
FIG. 9B illustrates a data group D11, among the data groups illustrated in FIG. 9A, which corresponds to a wavelength (first wavelength) of light emitted from a first light source 321 such that the data group D11 is plotted in a graph in which the vertical axis (Y axis) represents absorbances and the horizontal axis (X axis) represents elapse of time (seconds) from the start of output of detection information;
FIG. 10 illustrates an example of a structure of a message box MB1;
FIG. 11 illustrates an outline of training of a deep learning algorithm;

FIG. 12 illustrates an example of analysis using the deep learning algorithm;

FIG. 13 illustrates an example of a structure of a message box MB1';

FIG. 14 shows a flow of a re-analysis process executed by the controller 70;

FIG. 15 illustrates an example of output of a dialog box MB3 for causing an operator to input selection of a re-analysis;

FIG. 16 illustrates an example of a re-analysis item database DB3;

FIG. 17 illustrates an example of a structure of a message box MB1";

FIG. 18 illustrates an example of a re-analysis result database DB4;

FIG. 19A illustrates an example of an outer appearance of a training apparatus 5;

FIG. 19B illustrates an example of a hardware configuration of the training apparatus 5;

FIG. 20 is a flow chart showing another method for calculating a probability of occurrence of nonspecific reaction;

FIG. 21 illustrates an example of an S value distribution of a biological sample group for which nonspecific reaction does not occur;

FIG. 22 illustrates a histogram in which the horizontal axis represents a probability (0.0 to 1.0), of occurrence of nonspecific reaction, outputted from the deep learning algorithm, the horizontal axis is divided at intervals of 0.05, and the vertical axis represents a frequency of biological samples belonging to each divided range; and

FIG. 23 illustrates an ROC curve obtained as a result of an ROC analysis of the biological samples illustrated in FIG. 22.

## DETAILED DESCRIPTION

### 1. Analyzer 1

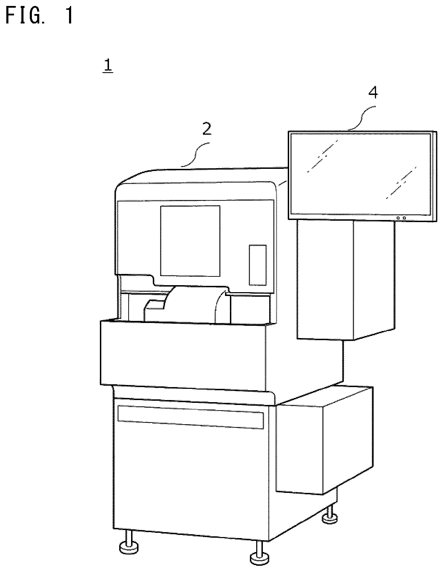

An analyzer of the present embodiment as defined in the appended claims (hereinafter, simply referred to as "analyzer 1") will be described with reference to FIG. 1 to FIG. 12.

The analyzer 1 measures a biological sample by turbidimetric immunoassay. The turbidimetric immunoassay is a measurement method for measuring a process of antigen-antibody reaction between an antigen or an antibody in a biological sample and an antibody or an antigen, in a measurement reagent, which specifically binds to the antigen or the antibody in the biological sample. The analyzer 1 analyzes, for example, D-dimer as a fibrin degradation product or FDP as a fibrin and/or fibrinogen degradation product.

In a case where D-dimer is measured, a measurement reagent containing carrier particles to which an antibody for recognizing D-dimer is fixed, can be used as a measurement reagent. Examples of the D-dimer measurement reagent include LIAS AUTO (registered trademark)·D-dimer NEO manufactured by SYSMEX CORPORATION, Nanopia (registered trademark) D-dimer manufactured by SEKISUI MEDICAL CO. LTD., and LPIA-GENESIS (registered trademark) D-dimer manufactured by LSI Medience Corporation.

In a case where FDP is measured, a measurement reagent containing carrier particles to which an antibody for recognizing FDP is fixed, can be used as a measurement reagent. Examples of the FDP measurement reagent include LIAS AUTO (registered trademark) P-FDP manufactured by SYSMEX CORPORATION, Nanopia (registered trademark) P-FDP manufactured by SEKISUI MEDICAL CO., LTD., and LPIA (registered trademark) FDP-P manufactured by LSI Medience Corporation.

The analyzer 1 may perform, for example, analysis of a biological sample by nephelometric immunoassay as well as measurement by turbidimetric immunoassay. The analyzer 1 may analyze not only D-dimer or FDP but also, for example, soluble fibrin monomer complex (hereinafter, may be abbreviated as "FMC"), von Willebrand factor antigen (hereinafter, may be abbreviated as "VWF: Ag"), immunoglobulins IgG, IgA, and IgM, complement markers C3 and C4, antistreptolysin-O, vancomycin, μ-albumin, prealbumin (P-Alb), lipoprotein (a), adenosine 5'-diphosphate (ADP), collagen, epinephrine, or CRP.

A biological sample measured by the analyzer 1 is plasma. The biological sample measured by the analyzer 1 may be a blood sample such as whole blood or serum, urine, pleural fluid, ascites, lymph, interstitial fluid, cerebrospinal fluid, and the like in addition to plasma.

### 1-1. Hardware configuration of analyzer 1

The analyzer 1 can prepare a measurement sample by adding, to a biological sample, a measurement reagent containing an antibody or an antigen that specifically binds (that is, causes antigen-antibody reaction with) to an antigen or an antibody in the biological sample, apply light to the prepared measurement sample, detect transmitted light from the measurement sample, and analyze the antigen or the antibody in the biological sample based on the detected light, to detect occurrence of nonspecific reaction in the measurement sample. FIG. 1 illustrates an example of an outer appearance of the analyzer 1. The analyzer 1 includes a measurement device 2 for applying light to a measurement sample for a predetermined time period, and obtaining detection information indicating an intensity of light transmitted through the measurement sample, and an input/output device 4 capable of performing data input and output.

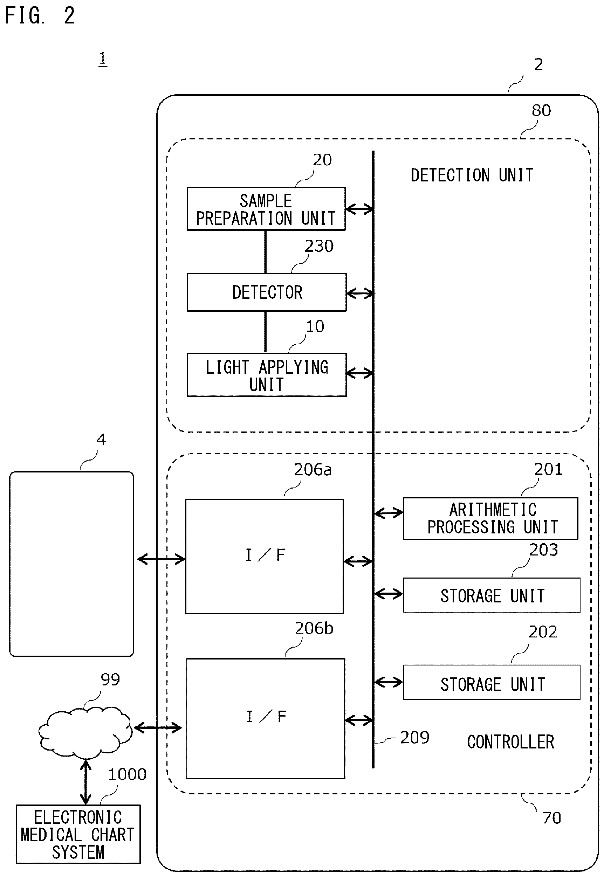

FIG. 2 illustrates an example of a hardware configuration of the analyzer 1. The measurement device 2 of the analyzer 1 includes a controller 70 and a detection unit 80. The controller 70 includes an arithmetic processing unit 201 for performing data processing, a storage unit 203 used as a work area for the data processing, a storage unit 202 for storing information to be transferred to the storage unit 203, a bus 209 for transmitting data between the units, and interfaces (I/Fs) 206a and 206b for performing data input/output with an external device. The controller 70 is connected to an electronic medical chart system 1000 via a network 99.

The arithmetic processing unit 201 is implemented by a CPU (central processing unit). The input/output device 4 is connected to the interface (I/F) 206a, and the network 99 is connected to the interface (I/F) 206b. The interface (I/F) 206a is a USB and the interface (I/F) 206b is Ethernet. The storage unit 203 is implemented by a DRAM and an SRAM. The storage unit 202 is implemented by a solid-state drive. The input/output device 4 is implemented by a touch-panel type display, and receives input from an operator by contact with the display and displays information on the display. Signals are transmitted via the bus 209 in the measurement device. The configuration of the controller 70 is not limited to the above-described one. For example, IEEE1394 may be used as the interface (I/F) 206a or the interface (I/F) 206b. For example, a hard disk may be used as the storage unit 202. The input/output device 4 may include a keyboard and/or a mouse as an input device and a liquid crystal display or an organic EL display as an output device.

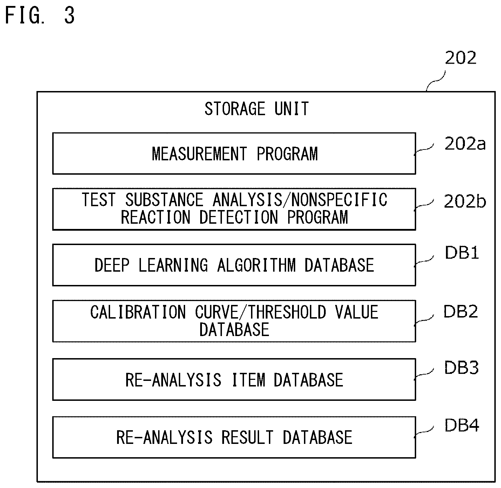

FIG. 3 illustrates information stored in the storage unit 202. The storage unit 202 stores: a measurement program 202a for causing the measurement device 2 to perform a measurement process and output detection information; a test substance analysis/nonspecific reaction detection program 202b for generating a data group from the detection information and analyzing the data group; a deep learning algorithm database DB1 for storing a deep learning algorithm used for analysis; a calibration curve/threshold value database DB2 for storing a calibration curve corresponding to each measurement item, a high concentration determination threshold value corresponding to each measurement item, and a nonspecific reaction determination threshold value corresponding to each measurement item; a re-analysis item database DB3 for storing information of items of a re-analysis performed when the re-analysis is needed because of suspicion of occurrence of nonspecific reaction; and a re-analysis result database DB4 for associating a data group about progress of antigen-antibody reaction for each biological sample for which presence or absence of occurrence of nonspecific reaction has been specified by the re-analysis, with identification information (sample No. or the like) of the biological sample, and presence or absence of occurrence of nonspecific reaction, and storing the data group associated with the identification information and the presence or absence of occurrence of nonspecific reaction.

Referring again to FIG. 2, the detection unit 80 includes a sample preparation unit 20, a light applying unit 10, and a detector 230.

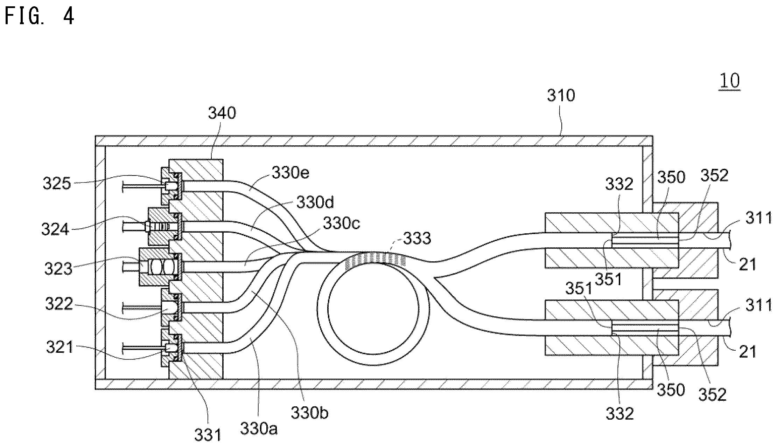

FIG. 4 illustrates a structure of the light applying unit 10. The light applying unit 10 includes five light sources 321, 322, 323, 324, and 325, five optical fiber sections 330a, 330b, 330c, 330d, and 330e disposed so as to correspond to the five light sources 321 to 325, and one holding member 340 for holding the light sources 321 to 325 and incident ends 331 of the optical fiber sections 330a to 330e. The light sources 321 to 325, the optical fiber sections 330a to 330e, and the holding member 340 are housed in a metal housing 310. Each of the light sources 321 to 325 is implemented by a LED. The first light source 321 emits 660 nm (first wavelength) light. The second light source 322 emits 405 nm (second wavelength) light. The third light source 323 emits 800 nm (third wavelength) light. The fourth light source 324 emits 340 nm (fourth wavelength) light. The fifth light source 325 emits 575 nm (fifth wavelength) light. The first light source 321 to the fifth light source 325 are controlled by the controller 70 so as to sequentially emit light one by one repeatedly such that each of the first light source 321 to the fifth light source 325 emits light for a predetermined time period (for example, T seconds (T is less than 0.1)) as described below, until a predetermined time (for example, 200 seconds) elapses since start of light application.

The optical fiber sections 330a to 330e are each implemented by a cable which has one core and is obtained by bundling optical fiber element wires. The optical fiber sections 330a to 330e are bundled into one at an intermediate section 333, and the optical fiber sections 330a to 330e bundled into one are divided into two bundles, and an emission end 332 of each bundle is held at an outlet 311 disposed in the housing 310. An incident end 352 of an optical fiber 21 that connects between the light applying unit 10 and the detector 230 is also held at each outlet 311. A homogenizing member 350 for homogenizing an intensity distribution of light emitted through the emission end 332 is disposed between the emission end 332 for the optical fiber sections 330a to 330e and the incident end 352 of the optical fiber 21. The homogenizing member 350 reflects therein light incident on an incident surface 351 multiple times, and is implemented by, for example, a polygonal-prism-shaped rod homogenizer.

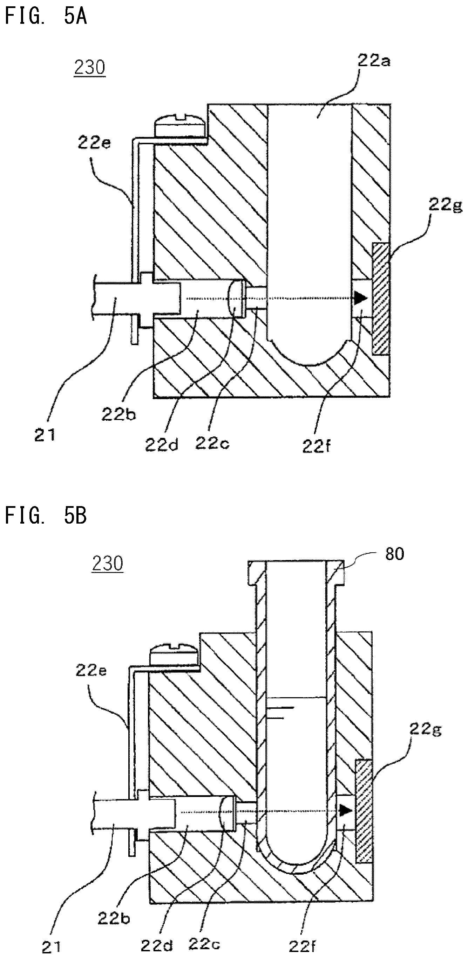

FIGS. 5A and 5B illustrate a structure of the detector 230. The detector 230 is disposed at each optical fiber 21 connected to the light applying unit 10. The structure for connection between the optical fiber 21 and the detector 230 and the hardware configuration of the detector 230 are common in all the detectors 230. Therefore, in the description herein, one detector 230 will be described. FIG. 5A illustrates the detector 230 in which a transparent container 15 for mixing a measurement reagent and a biological sample is not held. The detector 230 has a hole 22b into which the other end 353 of the optical fiber 21 having the incident end 352 (FIG. 4) held at the outlet 311 of the light applying unit 10 is inserted.

Furthermore, a connection hole 22c that connects the hole 22b to a holding portion 22a for holding the container 15 is formed in the detector 230.

The diameter of the hole 22b is larger than the diameter of the connection hole 22c. A lens 22d for condensing light from the optical fiber 21is disposed at the end portion of the hole 22b. Furthermore, a hole 22f is formed at an inner wall surface of the holding portion 22a so as to oppose the connection hole 22c. A light receiver 22g is disposed deeply in the hole 22f. The light receiver 22g is implemented by, for example, a photodiode, and outputs an electrical signal corresponding to an amount of received light. Light transmitted through the lens 22d is incident on a light receiving surface of the light receiver 22g through the connection hole 22c, the holding portion 22a, and the hole 22f. The optical fiber 21 is fixed by a plate spring 22e in a state where the end 353 is inserted in the hole 22b.

FIG. 5B illustrates the detector 230 that holds the container 15. By holding the container 15 in the holding portion 22a, light condensed by the lens 22d is incident on the light receiver 22g through the container 15 and the measurement sample contained in the container 15. When antigen-antibody reaction progresses in the measurement sample, a turbidity of the measurement sample is enhanced. According thereto, an amount (amount of transmitted light) of light transmitted through the measurement sample is reduced, and a level of an electrical signal outputted from the light receiver 22g is lowered. The electrical signal outputted by the light receiver 22g is converted to a digital signal by an A/D converter 22h, and transmitted via the I/F 206 (see FIG. 2) to the controller 70. The signal that is outputted by the light receiver 22g and converted by the A/D converter 22h represents digital data based on the amount of transmitted light. The detector 230 may be structured such that the light receiver 22g is disposed on a straight line intersecting a straight line that connects between the lens 22d and the light receiver 22g, and light scattered by the measurement sample is detected.

The sample preparation unit 20 dispenses a biological sample and a measurement reagent into the container 15, and transfers the container 15 that contains a measurement sample in which the biological sample and the measurement reagent are mixed, to the holding portion 22a of the detector 230.

As the light applying unit 10, the sample preparation unit 20, and the detector 230, for example, the units disclosed in U.S. Patent No. 10,048,249 can be used.

1-2. Analysis process (measurement/test substance analysis/nonspecific reaction detection process)

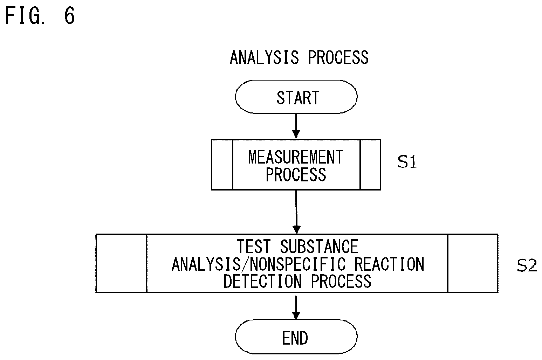

FIG. 6 shows a flow of an analysis process (measurement/test substance analysis/nonspecific reaction detection process) performed by the controller 70. The controller 70 executes a measurement process according to the measurement program 202a in step S1. Subsequently, the controller 70 executes a test substance analysis/nonspecific reaction detection process according to the test substance analysis/nonspecific reaction detection program 202b in step S2. The measurement process is started when the controller 70 receives a process start instruction inputted through the input/output device 4 by an operator.

1-3. Process according to measurement program

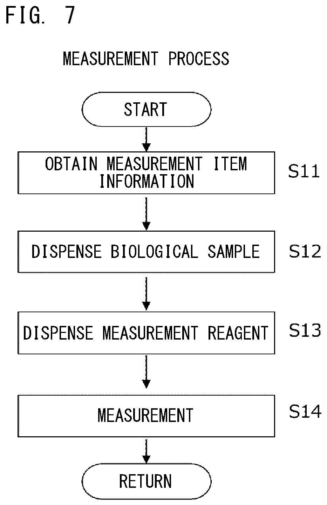

FIG. 7 shows a flow of the measurement process executed by the controller 70 according to the measurement program 202a. In step S11, the controller 70 obtains, for each biological sample, information of a measurement item ordered for the biological sample, based on the information inputted from the input/output device 4 by the operator. The controller 70 may obtain the information of the measurement item via the network 99 from, for example, the electronic medical chart system 1000 of a medical institution. The biological sample and the information of the measurement item can be associated with each other by, for example, an identifier issued when the test is requested.

The controller 70 causes the sample preparation unit 20 to dispense the biological sample into the container 15 in step S12. In step S13, the controller 70 causes the sample preparation unit 20 to dispense, into the container 15, a measurement reagent corresponding to the measurement item obtained in step S11 and prepare a measurement sample.

In step S14, the controller 70 causes the light applying unit 10 to start applying light to the container 15 in which the measurement sample has been prepared in step S13. Specifically, the first light source 321 to the fifth light source 325 sequentially emit light one by one repeatedly such that each of the first light source 321 to the fifth light source 325 emits light for a predetermined time period (for example, T seconds (T is less than 0.1)), until a predetermined time (for example, 200 seconds) elapses since start of light application. The detector 230 outputs electrical signals (digital data) corresponding to intensities (that is, intensities of transmitted light) of light received via the container 15 one by one such that each electrical signal is outputted for the predetermined time period (for example, T seconds). The digital data having been output indicates an intensity of light transmitted through the measurement sample. The outputted digital data set is transmitted as the detection information to the controller 70. Thus, each piece of data of the detection information expresses an intensity of the transmitted light at each measurement time point from start of light application to elapse of the predetermined time (for example, 200 seconds).

1-4. Process according to test substance analysis/nonspecific reaction detection program 202b

i. Test substance analysis/nonspecific reaction detection process

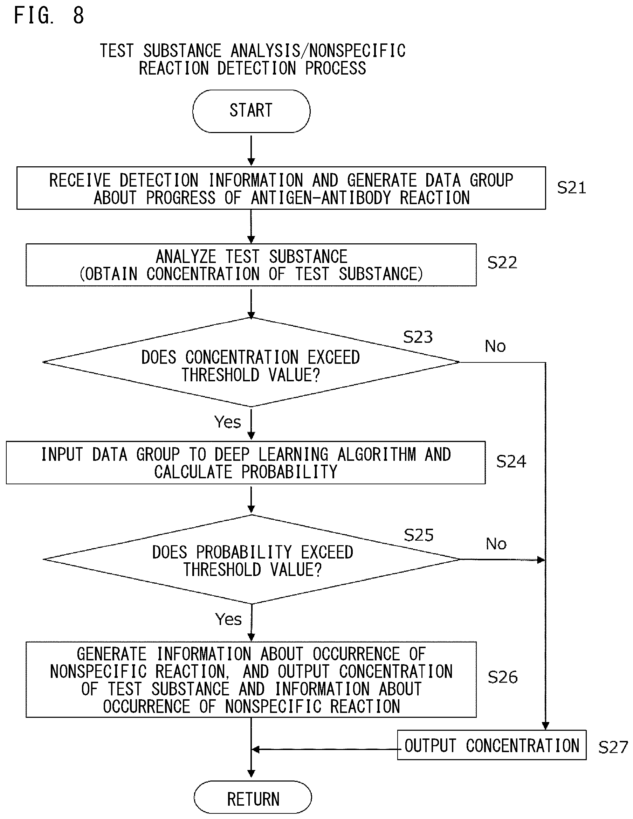

FIG. 8 shows a flow of the test substance analysis/nonspecific reaction detection process executed by the controller 70 according to the test substance analysis/nonspecific reaction detection program 202b.

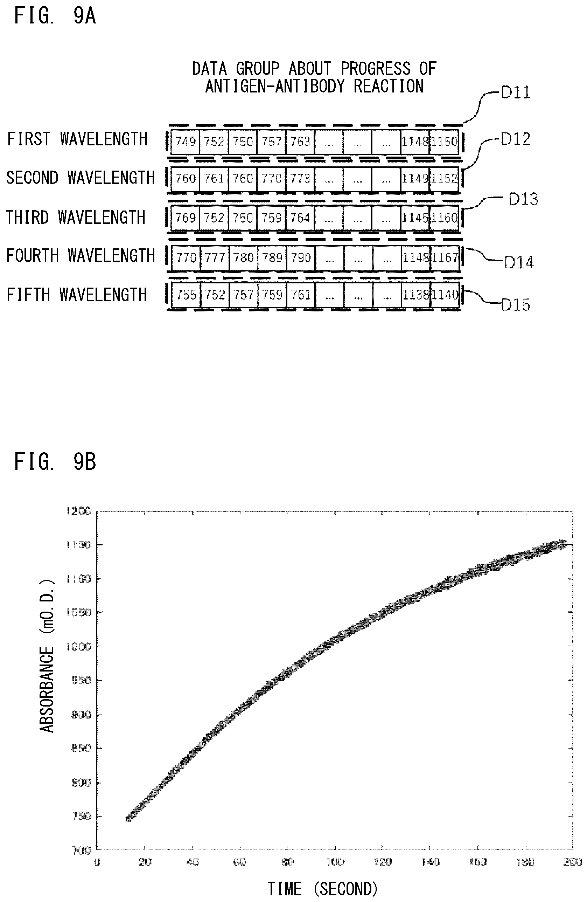

The controller 70 receives the detection information from the detector 230 and generates a data group about progress of antigen-antibody reaction from the received detection information, in step S21. Specifically, the controller 70 receives the detection information from the detector 230, classifies the received detection information for each of the first light source 321 to the fifth light source 325 (that is, for each of wavelengths of light emitted from the light sources), and stores the detection information in the storage unit 202, in step S21. The controller 70 converts each piece of digital data of the stored detection information to absorbance, chronologically arranges absorbances obtained by the conversion, and stores the absorbances as a data group about progress of antigen-antibody reaction in the storage unit 202. Since each piece of the digital data of the detection information represents an intensity of transmitted light, the detection information can be converted to absorbance by, for example, expressing a reciprocal of each piece of the digital data of the detection information by a common logarithm. FIG. 9A illustrates an example of the data groups about progress of antigen-antibody reaction. As illustrated in FIG. 9A, data groups D11 to D15 are generated for respective wavelengths of light emitted from the light sources and include absorbances arranged in the order of output from the detector 230. Each piece of data in the data group D11 expresses an absorbance at each measurement time point from the start of light application to elapse of a predetermined time (for example, 200 seconds). Similarly, each piece of data in the data groups D12 to D15 also expresses an absorbance at each measurement time point from the start of light application to elapse of the predetermined time (for example, 200 seconds).

FIG. 9B illustrates the data group D11, among the data groups illustrated in FIG. 9A, which corresponds to the wavelength (first wavelength) of light emitted from the first light source 321 such that the data group D11 is plotted in a graph in which the vertical axis (Y axis) represents absorbances and the horizontal axis (X axis) represents elapse of time (seconds) from the start of output of the detection information. When an antigen or an antibody as a test substance (hereinafter, an antigen or an antibody to be analyzed is referred to as "test substance") is in the biological sample, antigen-antibody reaction between the antigen or the antibody and an antibody or an antigen fixed to carrier particles progresses, whereby the carrier particles agglutinate, and turbidity of the measurement sample in the container 15 is enhanced. Therefore, when a test substance is in the biological sample, absorbance is enhanced according to the concentration.

The controller 70 analyzes the test substance in step S22 shown in FIG. 8. That is, the controller 70 obtains a concentration of the test substance in the biological sample, based on the data group (for example, data group D11) that corresponds to a wavelength according to the measurement item and a calibration curve stored in the calibration curve/threshold value database DB2. Specifically, the controller 70 calculates a change amount or change rate of the absorbance from the data group. The change amount or change rate of the absorbance can be calculated from the data group by using a Rate method or a VLin method. The Rate method is a method in which a start point and an end point are preset, and an absorbance change amount per one minute is calculated by linear regression, for a data group included between the start point and the end point. The VLin method is a method in which a start point and an end point at which an absorbance change amount is maximal and linear approximation is optimal, are set for a data group, and an absorbance change amount per one minute is calculated by linear regression, for the data group included between the start point and the end point. Whether calculation of the change amount or change rate is performed in the Rate method or the VLin method is determined by the controller 70 according to the measurement item. Before the change amount or change rate of the absorbance is calculated, the data group may be subjected to pretreatment such as smoothing, sharpness process, or caving.

The calibration curve is obtained in a manner in which a reference substance having a known concentration of a test substance is diluted at different dilution rates, each diluted reference substance is measured to obtain a change amount or change rate of absorbance, and each change amount or change rate is plotted in a graph in which the vertical axis represents change amounts or change rates of the absorbance and the horizontal axis represents concentrations of the test substance, and linear regression is performed. The controller 70 applies, to the calibration curve, the change amount or change rate of the absorbance obtained from the data group, and obtains the concentration of the test substance in the biological sample.

In step S23 shown in FIG. 8, the controller 70 determines whether or not the concentration obtained in step S22 exceeds a high concentration determination threshold value stored in the calibration curve/threshold value database DB2. In a case where the concentration of the test substance obtained in step S22 is less than or equal to the threshold value ("No" in step S23), the process is advanced to step S27 to output the concentration of the test substance obtained in step S22 to the input/output device 4, and the process is ended. In a case where the concentration obtained in step S22 exceeds the threshold value ("Yes" in step S23), the controller 70 advances the process to step S24. The controller 70 performs detection of occurrence of nonspecific reaction in the measurement sample in steps S24 and S25.

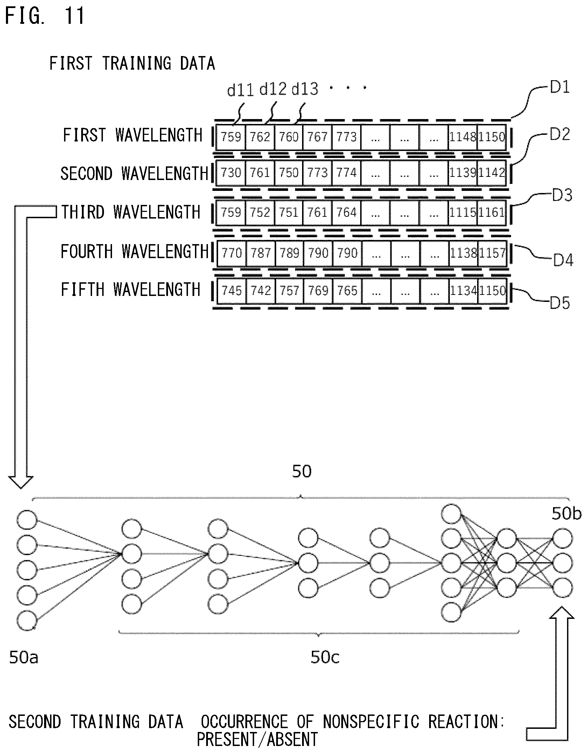

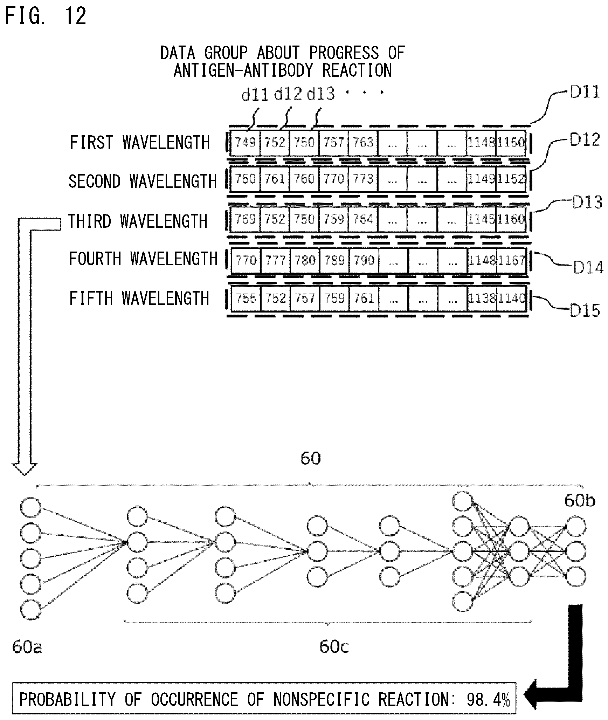

A method for generating a deep learning algorithm 60 illustrated in FIG. 12 will be described. The deep learning algorithm 60 is not limited to any specific one as long as the deep learning algorithm 60 is an algorithm having a neural network structure. The deep learning algorithm 60 may include, for example, a convolutional neural network, a full-connect neural network, and combinations thereof. The deep learning algorithm 60 is generated by training an untrained deep learning algorithm 50 (FIG. 11). FIG. 11 illustrates an outline of training of the deep learning algorithm. The training of the deep learning algorithm is performed by a training apparatus 5 described below.

For training data for training the deep learning algorithm 50, a data group, about progress of antigen-antibody reaction, obtained from a biological sample for which whether or not nonspecific reaction has occurred is known, is used as first training data. The first training data can be generated in the method described in step S21 shown in FIG. 8. As illustrated in FIG. 11, the first training data includes a first data group D1 corresponding to a first wavelength, a second data group D2 corresponding to a second wavelength, a third data group D3 corresponding to a third wavelength, a fourth data group D4 corresponding to a fourth wavelength, and a fifth data group D5 corresponding to a fifth wavelength. The data group D1 includes a plurality of data pieces d11, d12, d13··· each representing an absorbance. Similarly, the data group D2 to the data group D5 each include a plurality of data pieces each representing an absorbance. The first training data is inputted to an input layer 50a of the neural network 50 illustrated in FIG. 11. A label ("presence/absence of occurrence of nonspecific reaction" in the example illustrated in FIG. 11) that indicates presence or absence of occurrence of nonspecific reaction and corresponds to the inputted first training data is inputted as second training data to 50b as an output layer. Through these inputs, the first training data and the second training data are associated with each other for each biological sample, and a weight for each layer in an intermediate layer 50c in the neural network 50 is calculated. Input of the first training data and the second training data, and calculation of the weight of each layer in the intermediate layer 50c are performed for multiple biological samples, thereby generating the deep learning algorithm 60, illustrated in FIG. 12, having been trained. A softmax function is applied to the output layer 60b of the deep learning algorithm 60. Thus, a value representing a probability of occurrence of nonspecific reaction is outputted from the deep learning algorithm 60.

With reference to FIG. 12, in step S24 shown in FIG. 8, the controller 70 operates to input the data group D11, obtained in step S21, corresponding to the wavelength (first wavelength) of light emitted from the first light source 321, the data group D12, obtained in step S21, corresponding to the wavelength (second wavelength) of light emitted from the second light source 322, the data group D13, obtained in step S21, corresponding to the wavelength (third wavelength) of light emitted from the third light source 323, the data group D14, obtained in step S21, corresponding to the wavelength (fourth wavelength) of light emitted from the fourth light source 324, and the data group D15, obtained in step S21, corresponding to the wavelength (fifth wavelength) of light emitted from the fifth light source 325, to the input layer 60a of the deep learning algorithm 60 stored in the deep learning algorithm database DB1. In the deep learning algorithm 60, the inputted data groups D11 to D15 are processed at the intermediate layer 60c, and a probability (98.4% in the example illustrated in FIG. 12) of occurrence of nonspecific reaction is outputted from the output layer 60b. The value representing the probability may not necessarily be indicated by a percentage, and may be indicated by a relative value. In the description herein, information based on a value representing a probability such as a percentage and a relative value is referred to as information indicating probability.

The "probability of occurrence of nonspecific reaction" represents a probability that nonspecific reaction has actually occurred in a measurement sample to be analyzed. For example, this means that, in a case where the probability of occurrence of nonspecific reaction is 80%, a probability that the nonspecific reaction has actually occurred in the measurement sample is 80%. In other words, the probability of occurrence of nonspecific reaction being 80% means that, in a case where the number of measurement samples for which the probability of occurrence of the nonspecific reaction is 80% is 100, the number of the measurement samples in which the nonspecific reaction has actually occurred is 80, and the nonspecific reaction has not occurred in the remaining 20 samples.

As illustrated in FIG. 11 and FIG. 12, the structure of the data group in the first training data and the structure of the data group obtained in step S21 are preferably the same. However, the present invention is not limited thereto. For example, although the first wavelength to the fifth wavelength in the first training data are the same as the first wavelength to the fifth wavelength in the data group obtained in step S21, only a part of wavelengths may be used in the data group in the first training data and/or the data group obtained in step S21. Furthermore, although the number of pieces of data in each data group in the first training data and the number of pieces of data in each data group in the data groups obtained in step S21 are equal to each other, the number of pieces of data in each data group in the first training data may be different from the number of pieces of data of each data group in the data groups obtained in step S21.

The structure of the data group in the first training data and the data group obtained in step S21 may not necessarily be obtained for a plurality of wavelengths, and may be obtained for one wavelength, and the obtained data group may be inputted to the deep learning algorithm 50 or the deep learning algorithm 60.

The deep learning algorithm 60 may be generated so as to be commonly used for a plurality of measurement items, or may be generated for each measurement item. In a case where the deep learning algorithm 60 is generated for each measurement item, the controller 70 selects, in step S24 shown in FIG. 8, the deep learning algorithm 60 to which the data group is to be inputted, from a plurality of deep learning algorithms, according to the measurement item obtained in step S11.

In step S25 shown in FIG. 8, the controller 70 determines whether or not the probability, of occurrence of nonspecific reaction, outputted from the deep learning algorithm 60 in step S24 exceeds the nonspecific reaction determination threshold value stored in the calibration curve/threshold value database DB2. In a case where the probability, outputted in step S24, of occurrence of nonspecific reaction is less than or equal to the threshold value ("No" in step S25), the process is advanced to step S27 to output the concentration of the test substance obtained in step S22 to the input/output device 4, and the process is ended. In a case where the probability, outputted in step S24, of occurrence of nonspecific reaction exceeds the threshold value ("Yes" in step S25), the controller 70 advances the process to step S26. The controller 70 operates to output the concentration obtained in step S22 to the input/output device 4 in step S26. Furthermore, the controller 70 generates information about occurrence of nonspecific reaction and outputs the information to the input/output device 4 in step S26.

The deep learning algorithm 60 may be generated so as to output a probability that nonspecific reaction has not occurred, from the output layer 60b. In this case, the controller 70 determines whether or not a probability, outputted from the deep learning algorithm 60 in step S24, that nonspecific reaction has not occurred is less than the nonspecific reaction determination threshold value stored in the calibration curve/threshold value database DB2. In a case where the probability, outputted in step S24, that nonspecific reaction has not occurred is less than the threshold value, the controller 70 advances the process to step S27. In a case where the probability is greater than or equal to the threshold value, the controller 70 advances the process to step S26.

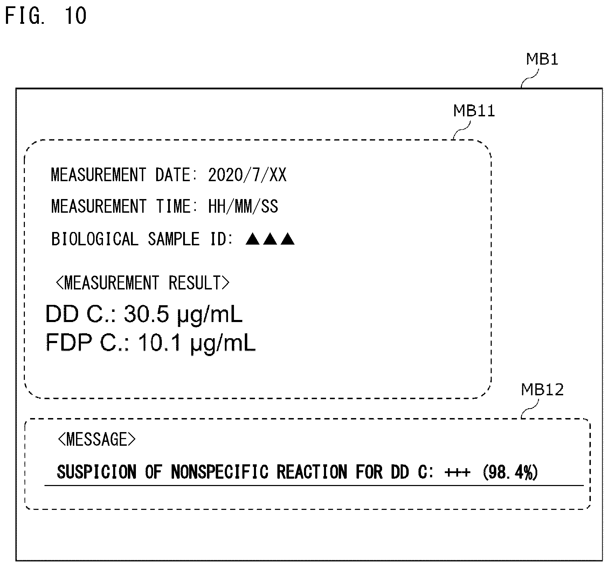

FIG. 10 illustrates an example of a message box MB1 for outputting information, about occurrence of nonspecific reaction, which is generated in step S26 and outputted to the input/output device 4. The message box MB1 includes a measurement item information region MB11 that indicates measurement item information including a measurement date, a measurement time, a biological sample identifier (biological sample ID), a measurement result, and the like, and a message region MB12 indicating information about occurrence of nonspecific reaction for the measurement result. As the measurement result in the measurement item information region MB11, the concentration, of the test substance as a measurement target, which is obtained in step S22 shown in FIG. 8 is displayed. In the example illustrated in FIG. 10, a D-dimer concentration (DD C) and an FDP concentration (FDP C) are indicated. In the message region MB12, the measurement item (DD C), an output label (+++) for qualitatively indicating suspicion of nonspecific reaction, and information (98.4%) indicating the probability are indicated. The information indicating the probability represents a probability, of occurrence of nonspecific reaction, outputted from the deep learning algorithm 60 in step S24. As the output label, "+", "++", "+++", and the like are used according to a magnitude of difference between the probability and the nonspecific reaction determination threshold value. Instead of "+", "++", and "+++", "low", "medium", "high", and the like may be used.

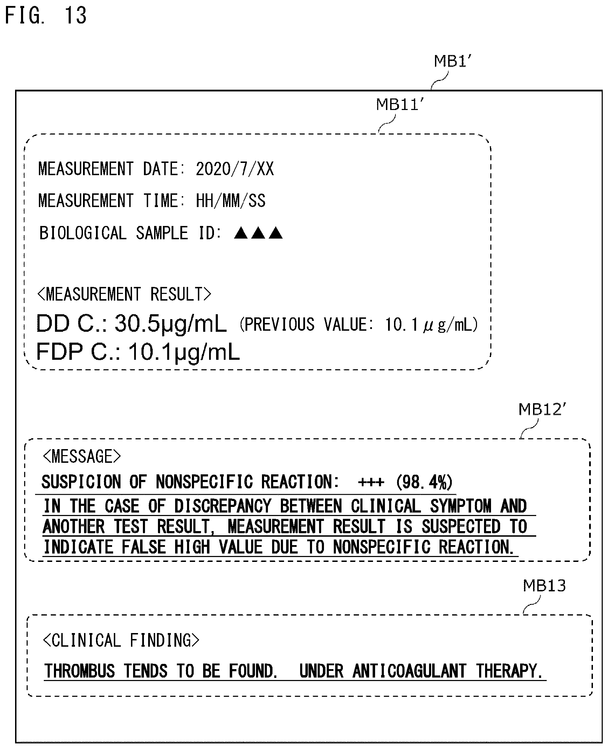

FIG. 13 illustrates a message box MB1' as a modification of the message box MB1. In the message box MB1', for a measurement item for which the probability of occurrence of nonspecific reaction exceeds the threshold value, the previous measurement result of the same subject is displayed in the measurement item information region MB11'. The previous measurement result of the same subject can be obtained from the electronic medical chart system 1000 by using a biological sample ID as a key. In the measurement item information region MB11', for a measurement item for which a probability of occurrence of nonspecific reaction has exceeded the threshold value, a history of the previous measurement results or a previous measurement result for a measurement item different from the current measurement item may be displayed. In the message box MB1', in a case where there is a discrepancy between the concentration of a test substance obtained in step S22, and a concentration of another test substance or clinical information of the subject, a message indicating that the concentration displayed in the measurement item information region MB11' is suspected to have a false high value due to nonspecific reaction, is displayed in the message region MB12'. Thus, an operator can clearly know that a re-analysis is necessary. In the message box MB1', clinical information of the same subject is displayed in a clinical information region MB13. The clinical information can be obtained from the electronic medical chart system 1000 by using the biological sample ID as a key. The clinical information is obtained by diagnosing a subject, and includes a CT scan result, an MRI scan result, an ultrasonography result, an angiography result, a medication history, and the like. A treatment history includes information of a medication history indicating, for example, whether or not an anticoagulant agent is used.

In the measurement item information region MB11 and/or the measurement item information region MB11', a measurement result of a measurement item for which the probability of occurrence of nonspecific reaction exceeds the threshold value may not be displayed. Furthermore, in the measurement item information region MB11 and/or the measurement item information region MB11', a measurement result of an measurement item for which the probability of occurrence of nonspecific reaction exceeds the threshold value may be displayed with a label indicating that the result is indicated as a reference value or a value that is required to be confirmed. The label may be text representing "reference value", "confirmation is required", or the like, or a symbol such as "*" and "!".

The process step of step S24 shown in FIG. 8 may be performed also in a case where the concentration of the test substance obtained in step S22 does not exceed the threshold value ("No" in step S23). In a case where the process step of step S24 is not performed when the concentration of the test substance does not exceed the threshold value, a processing speed of the controller 70 can be inhibited from being reduced due to increase of an arithmetic amount. Meanwhile, in a case where the process step of step S24 is performed also when the concentration of the test substance does not exceed the threshold value, occurrence of nonspecific reaction can be more assuredly detected.

ii. Re-analysis process

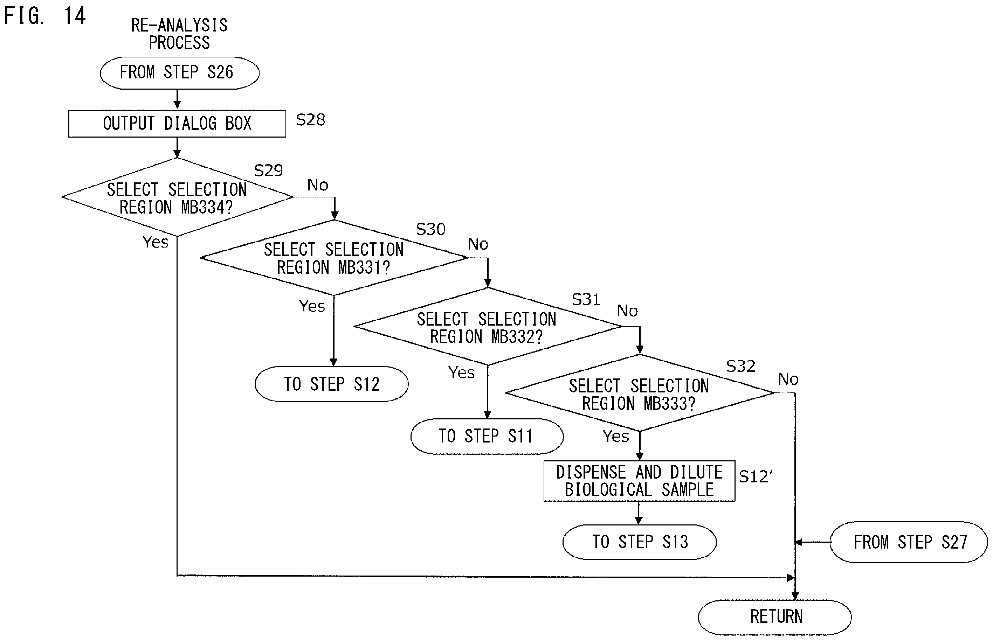

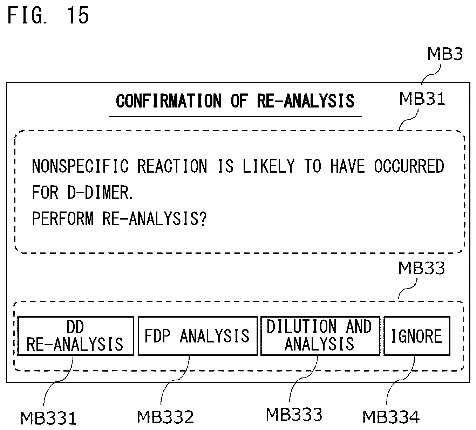

The analyzer 1 may be structured so as to perform a re-analysis process described below. In a case where the analyzer 1 is structured so as to perform a re-analysis process, the controller 70 executes a re-analysis process shown in FIG. 14 after step S26 shown in FIG. 8. The controller 70 operates to output a dialog box MB3 for causing an operator to input selection of a re-analysis, to the input/output device 4, in step S28. As illustrated in FIG. 15, the dialog box MB3 includes a message region MB31 including a message for inquiring of an operator whether or not a re-analysis is to be performed, and a selection region MB33 for causing the operator to select a re-analysis item. The selection region MB33 includes a selection region MB331 for selecting a re-analysis of the same measurement item as the measurement item (D-dimer in the example illustrated in FIG. 15) for which nonspecific reaction is likely to have occurred, a selection region MB332 for selecting an analysis of a measurement item (FDP in the example illustrated in FIG. 15) different from the measurement item (D-dimer in the example illustrated in FIG. 15) for which nonspecific reaction is likely to have occurred, a selection region MB333 for selecting a re-analysis performed by diluting a biological sample for the same measurement item as the measurement item (D-dimer in the example illustrated in FIG. 15) for which nonspecific reaction is likely to have occurred, and a selection region MB334 for performing selection so as not to perform a re-analysis.

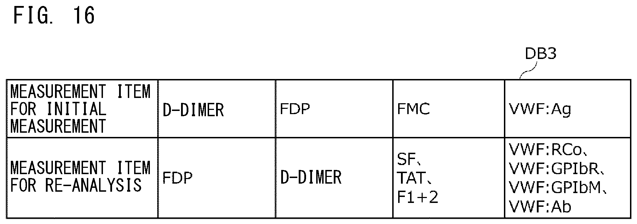

The measurement item displayed in the selection region MB332 is stored in the re-analysis item database DB3. As illustrated in FIG. 16, in the re-analysis item database DB3, a measurement item for an initial measurement and a measurement item for a re-analysis are stored so as to be associated with each other. The measurement item for the re-analysis is preferably an item related to the measurement item for the initial measurement. For example, in a case where the measurement item for the initial measurement is a thrombus·fibrinolysis-related measurement item, a thrombus·fibrinolysis-related measurement item different from the measurement item for the initial measurement is preferably selected as the measurement item for the re-analysis. In FIG. 16, in a case where the measurement item for the initial measurement is D-dimer, the measurement item for the re-analysis is FDP. In a case where the measurement item for the initial measurement is FDP, the measurement item for the re-analysis is D-dimer. In a case where the measurement item for the initial measurement is FMC, the measurement item for the re-analysis is soluble fibrin (SF), thrombin-antithrombin III complex (TAT), and/or prothrombin fragment 1+2 (F1+2). In a case where the measurement item for the initial measurement is VWF:Ag, the measurement item for the re-analysis is von Willebrand factor activity (ristocetin cofactor) (VWF:RCo), VWF binding ability (VWF:GPIbR) to recombinant GPIb in the presence of ristocetin, VWF binding ability (VWF:GPIbM) to recombinant GPIb mutant (gain-of-function), and/or monoclonal antibody binding ability (VWF:Ab) to A1 domain of VWF. In a case where a plurality of measurement items for the re-analysis correspond to the measurement item for the initial measurement, the controller 70 causes the input/output device 4 to display the selection regions MB332 of the respective measurement items one by one.

The controller 70 determines whether or not the selection region MB334 is selected by an operator, in step S29 shown in FIG. 14. In a case where the selection region MB334 is selected ("Yes" in step S29), the controller 70 returns the process to the test substance analysis/nonspecific reaction detection process shown in FIG. 8. In a case where the selection region MB334 is not selected ("No" in step S29), the controller 70 advances the process to step S30. The controller 70 determines in step S30 whether or not the selection region MB331 is selected. In a case where the selection region MB331 is selected ("Yes" in step S30), the controller 70 advances the process to step S12 shown in FIG. 7, and executes a measurement process for the measurement item obtained in step S11. In a case where the selection region MB331 is not selected ("No" in step S30), the controller 70 advances the process to step S31. The controller 70 determines in step S31 whether or not the selection region MB332 is selected. In a case where the selection region MB332 is selected ("Yes" in step S31), the controller 70 advances the process to step S11 shown in FIG. 7, obtains a measurement item (FDP in the example illustrated in FIG. 15) designated in the selection region MB332, and executes a measurement process for the measurement item. In a case where the selection region MB332 is not selected ("No" in step S31), the controller 70 advances the process to step S32. The controller 70 determines in S32 whether or not the selection region MB333 is selected. In a case where the selection region MB333 is selected ("Yes" in step S32), the controller 70 advances the process to step S12' to cause the sample preparation unit 20 to dispense the biological sample into the container 15 and dilute the biological sample. Subsequently, the controller 70 advances the process to step S13 shown in FIG. 7, and executes a measurement process for the measurement item obtained in step S11. In a case where the selection region MB333 is not selected ("No" in step S32), the controller 70 returns the process to the test substance analysis/nonspecific reaction detection process shown in FIG. 8. In a case where the process is advanced to step S27 in the test substance analysis/nonspecific reaction detection process shown in FIG. 8 ("No" in step S23 or step S25), the controller 70 does not execute the re-analysis process.

Selection from the selection region MB331 to the selection region MB334 may not necessarily be performed by an operator, and may be performed by the controller 70 according to initial setting or setting performed by an operator before start of the measurement. Furthermore, the controller 70 may select from the selection region MB331 to the selection region MB334 according to a probability, outputted in step 24 shown in FIG. 8, of occurrence of nonspecific reaction.

In the re-analysis for the same measurement item as the measurement item for the initial measurement, a measurement reagent in the same vial as for the initial measurement may be used or a measurement reagent in a vial different from the vial for the initial measurement may be used. In a case where a measurement reagent in a vial different from that for the initial measurement is used, a measurement reagent in another vial in the same lot as for the initial measurement may be used, or a measurement reagent in another vial in a lot different from a lot for the initial measurement may be used. Furthermore, in a case where a measurement reagent in a vial different from that for the initial measurement is used, a measurement reagent, for the same measurement item, which has been developed and manufactured by a company different from a company for the initial measurement, may be used.

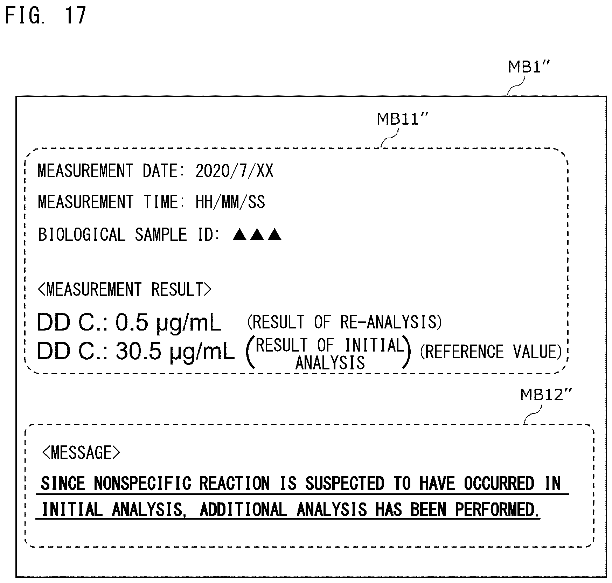

FIG. 17 illustrates an example of a message box MB1" displayed by the input/output device 4 in the re-analysis process, in step S27 shown in FIG. 8. In the message box MB1", the concentration obtained in the re-analysis, the concentration obtained in the initial measurement, and a message indicating that the concentration obtained in the initial measurement indicates a reference value, are displayed in the measurement item information region MB11". In the message region MB12", a message indicating that the displayed information is related to a result of the re-analysis, is displayed.

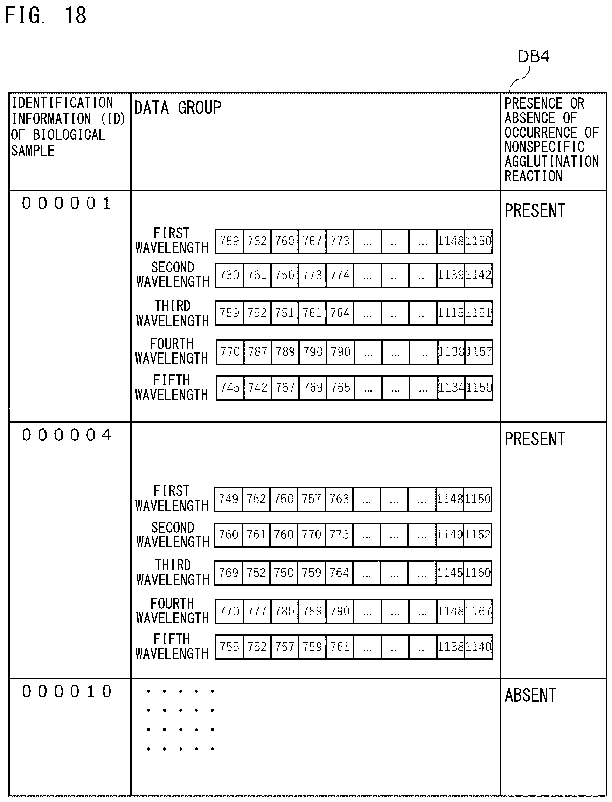

In a case where an operator specifies presence or absence of occurrence of nonspecific reaction by the re-analysis, the operator inputs information about presence or absence of occurrence of nonspecific reaction from the input/output device 4. The controller 70 may associate the inputted information about presence or absence of occurrence of nonspecific reaction, with the identification information of the biological sample and a data group, about progress of antigen-antibody reaction, obtained in step S21 shown in FIG. 8 for the biological sample, and store them in the re-analysis result database DB4 of the storage unit 202. FIG. 18 illustrates an example of the re-analysis result database DB4. The information stored in the re-analysis result database DB4 can be used as original data of the first training data and the second training data for further training the deep learning algorithm 60, and an analysis accuracy of the deep learning algorithm 60 can be further enhanced.

2. Training apparatus

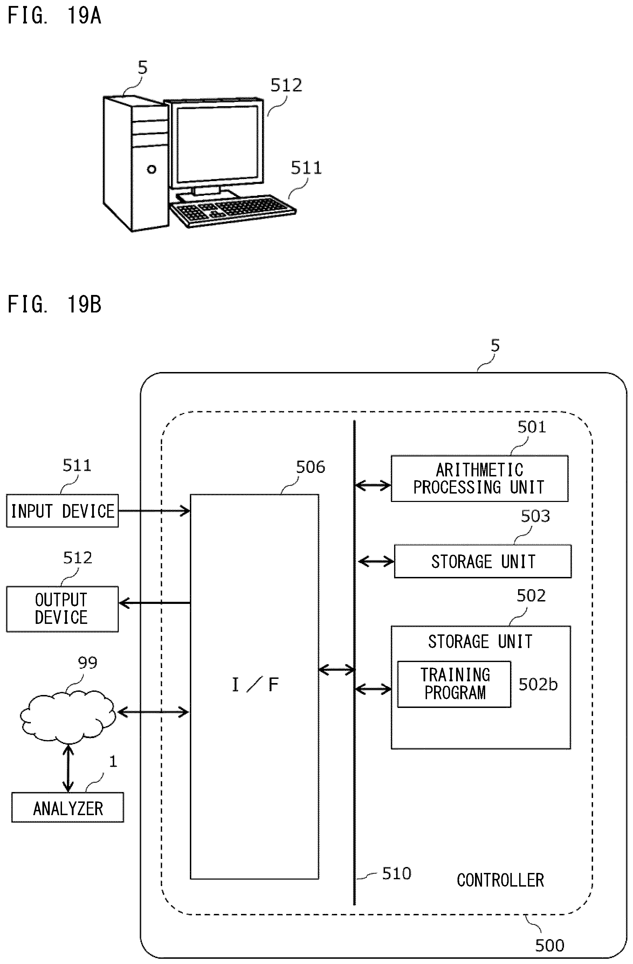

FIG. 19A illustrates an example of an outer appearance of the training apparatus 5 (hereinafter, simply referred to as "training apparatus 5") for training the deep learning algorithm 50. The training apparatus 5 is connected to an input device 511 and an output device 512 so as to be able to communicate therewith. A general-purpose computer can be used as the training apparatus 5.

FIG. 19B illustrates an example of a hardware configuration of the training apparatus 5. The training apparatus 5 includes a controller 500. The controller 500 includes an arithmetic processing unit 501, a storage unit 502, a storage unit 503, an interface (I/F) 506, and a bus 510. The arithmetic processing unit 501 is implemented by, for example, a CPU (central processing unit). The input device 511, the output device 512, and the network 99 are connected to the interface (I/F) 506. The interface (I/F) 506 is implemented by, for example, a USB, IEEE1394, or Ethernet. The storage unit 502 is implemented by, for example, a solid-state drive or a hard disk drive. The storage unit 502 stores a training program 502b for performing training of the deep learning algorithm 50 as described above. The storage unit 503 is implemented by, for example, a DRAM or an SRAM. The input device 511 is implemented by, for example, a keyboard or a mouse. The output device 512 is implemented by, for example, a liquid crystal display or an organic EL display. Signals are transmitted via the bus 510 in the training apparatus 5.

The training apparatus 5 is connected to the analyzer 1 over the network 99. Thus, the deep learning algorithm 60 having been trained can be transmitted to the analyzer 1 over the network 99, and information stored in the re-analysis result database DB4 of the analyzer 1 can be received from the analyzer 1 to further train the deep learning algorithm 60.

3. Modification

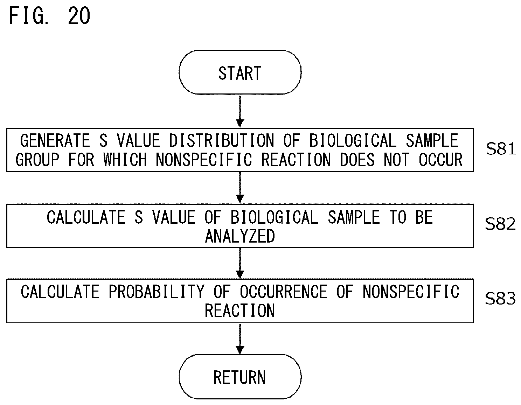

Information about occurrence of nonspecific reaction in antigen-antibody reaction can be obtained in a method described below in addition to or instead of the method for obtaining the information by using the deep learning algorithm 60 as described above. That is, in the method according to the modification, as shown in FIG. 20, the controller 70 generates, based on a predetermined function, a distribution of calculated values (S values) obtained from the function for data groups, about progress of antigen-antibody reaction, obtained from a plurality of biological samples for which nonspecific reaction does not occur, in step S81. In step S82, the controller 70 acquires, based on a predetermined function, a calculated value (S value) obtained from the function for a data group, about progress of antigen-antibody reaction, obtained from a biological sample to be analyzed. In step S83, the controller 70 obtains information about occurrence of nonspecific reaction based on the distribution generated in step S81 and the calculated value obtained in step S82 for the biological sample to be analyzed.

Specifically, in step S81, the controller 70 executes the process step of step S1 shown in FIG. 6 and the process step of step S21 shown in FIG. 8 for a biological sample for which nonspecific reaction does not occur, and obtains the data group about progress of the antigen-antibody reaction. The controller 70 calculates the S value according to the following mathematical expression 1 based on the obtained data group.

[Math. 1]

$$S = \frac{\alpha_1}{\text{maximum value of absorbance} - \text{minimum value thereof}} \quad (1)$$

(In expression (1), $\alpha_1$ represents an amplitude of a waveform rendered by the data group about progress of the antigen-antibody reaction);

[Math. 2]

$$f(x) = \alpha_1 \left(1 - \left(\frac{1}{1+e^{-\beta_1(x-t_1)}}\right)\right) + \gamma_1 \quad (2)$$

(In expression (2), $\alpha_1$, $\beta_1$, $\gamma_1$, and $t_1$ represent an amplitude, a wavelength inclination, y-axis direction transfer, and x-axis direction transfer, respectively, of the waveform rendered by the data group about progress of antigen-antibody reaction, for progress of the antigen-antibody reaction.)

$\alpha_1$ is determined by fitting of expression (2) to the obtained data group about progress of the antigen-antibody reaction. $\alpha_1$ in expression (2) determined in this manner is substituted into expression (1). The maximum value and the minimum value of absorbance included in expression (1) are determined from the obtained data group about progress of the antigen-antibody reaction. The S value is calculated according to expression (1) based on these values.

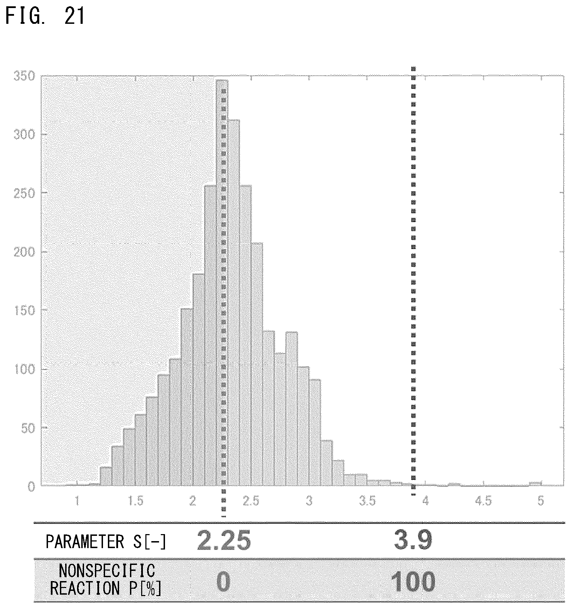

The controller 70 calculates the S values similarly for the other multiple biological samples for which nonspecific reaction does not occur, and generates a histogram in which the vertical axis represents the number of biological samples and the horizontal axis represents the S values, as illustrated in FIG. 21. At the most frequent value (S value=2.25 in the example illustrated in FIG. 21) in the S value distribution, a possibility of occurrence of nonspecific reaction is 0%, and, at the maximum value (S value=3.9 in the example illustrated in FIG. 21) of the S value, the possibility of occurrence of nonspecific reaction is 100%.

In step S82, the controller 70 calculates the S value based on the function of mathematical expression 1 for the data group, about progress of antigen-antibody reaction, obtained from the biological sample to be analyzed. In step S83, the controller 70 converts the S value obtained in step S82 to a probability of occurrence of nonspecific reaction in the biological sample to be analyzed. The S value can be converted to the probability of occurrence of nonspecific reaction, by, for example, assigning the S value to a linear regression expression generated from the most frequent value·probability (=0%) in the S value distribution and the maximum value·probability (=100%) in the S value distribution.

4. Verification of effect

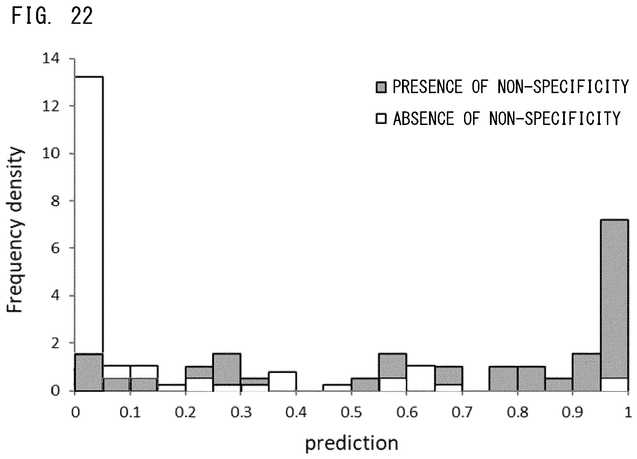

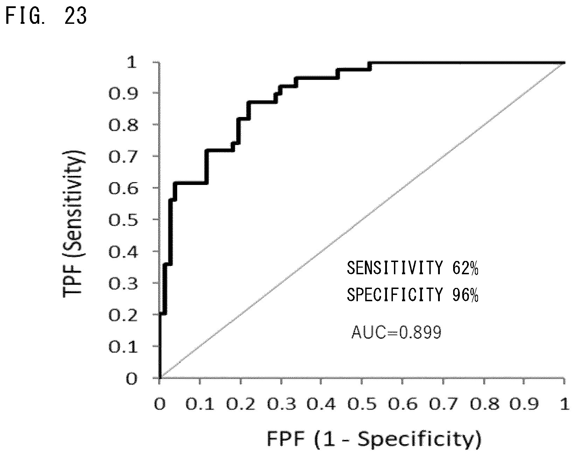

In measurement of D-dimer, presence or absence of occurrence of nonspecific reaction was confirmed by an immunoglobulin absorption test. A prediction accuracy in the analysis method of the present embodiment was evaluated by using 39 biological samples for which occurrence of nonspecific reaction was confirmed by the immunoglobulin absorption test, and 77 biological samples for which occurrence of nonspecific reaction was not confirmed by the immunoglobulin absorption test. In the deep learning algorithm, a data group set composed of the data group D11 corresponding to the first wavelength, the data group D12 corresponding to the second wavelength, the data group D13 corresponding to the third wavelength, and the data group D15 corresponding to the fifth wavelength was inputted. FIG. 22 and FIG. 23 illustrate the result thereof. FIG. 22 illustrates a histogram in which the horizontal axis represents a probability (0.0 to 1.0), of occurrence of nonspecific reaction, outputted from the deep learning algorithm, the horizontal axis is divided at intervals of 0.05, and the vertical axis represents a frequency of the biological samples belonging to each divided range. The height of the gray bar indicates a frequency of biological samples for which occurrence of nonspecific reaction was confirmed by the immunoglobulin absorption test, and the height of the white bar indicates a frequency of biological samples for which occurrence of nonspecific reaction was not confirmed by the immunoglobulin absorption test. FIG. 23 illustrates an ROC curve obtained as a result of an ROC analysis of the biological samples illustrated in FIG. 22.

The result of the ROC analysis indicated AUC=0.899. For example, in a case where the nonspecific reaction determination threshold value was 0.65, occurrence of nonspecific reaction was able to be predicted with a sensitivity of 62% and a specificity of 96%. These results indicate that occurrence of nonspecific reaction can be detected according to the present invention.

**Claims**

1.  A computer-implemented detection method for detecting occurrence of nonspecific reaction in analysis for an antigen or an antibody contained in a biological sample with use of a measurement reagent containing an antibody or an antigen that causes antigen-antibody reaction with the antigen or the antibody in the biological sample, the detection method comprising:
    generating a data group about progress of antigen-antibody reaction between the antigen or the antibody contained in the biological sample and the antibody or the antigen contained in the measurement reagent;

    inputting the data group to a deep learning algorithm; and
    generating information about occurrence of nonspecific reaction, based on a result outputted by the deep learning algorithm, wherein
    the generating of the data group comprises receiving detection information indicating an intensity of detected light that is detected through a measurement sample to which light is applied for a predetermined time period, and generating the data group from the detection information having been received, wherein each piece of data included in the data group expresses an intensity of the detected light that is detected at each measurement time point in the predetermined time period.

2.  The detection method of claim 1, wherein each piece of data included in the data group indicates an intensity of light transmitted through the measurement sample, an intensity of light absorbed by the measurement sample, or an intensity of light scattered by the measurement sample.

3.  The detection method of claim 1 or 2, wherein

    the detection information indicates an intensity of the detected light that is detected through the measurement sample to which a plurality of kinds of lights having different wavelengths are sequentially applied,
    the generating of the data group comprises generating a plurality of the data groups corresponding to the lights, respectively, based on the detection information, and
    the inputting of the data group to the deep learning algorithm comprises inputting a plurality of the data groups to the deep learning algorithm.

4.  An analysis method for analyzing an antigen or an antibody contained in a biological sample by using a measurement reagent containing an antibody or an antigen that causes antigen-antibody reaction with the antigen or the antibody in the biological sample, the analysis method comprising:

    preparing a measurement sample containing the biological sample and the measurement reagent;
    analyzing the antigen or the antibody contained in the biological sample; and
    performing the detection method of claim 1 for detecting occurrence of the nonspecific reaction.

5.  The analysis method of claim 4, further comprising

    applying light to the measurement sample for a predetermined time period and outputting detection information indicating an intensity of detected light that is detected through the measurement sample, wherein
    the analyzing of the antigen or the antibody contained in the biological sample comprises analyzing the antigen or the antibody based on the detection information.

6.  The analysis method of claim 5, wherein

    the generating of the data group comprises
    generating the data group from the detection information, and
    the analyzing of the antigen or the antibody contained in the biological sample comprises
    analyzing the antigen or the antibody based on the data group generated from the detection information.

7.  The analysis method of any one of claims 4 to 6, wherein, when a result of the analysis of the antigen or the antibody contained in the biological sample satisfies a first condition, the detection method for detecting occurrence of the nonspecific reaction is performed.

8.  The analysis method of any one of claims 4 to 7, wherein, when a result outputted from the deep learning algorithm satisfies a second condition, information about occurrence of the nonspecific reaction is outputted.

9. The analysis method of claim 8, wherein
the outputting of the information about occurrence of the nonspecific reaction further comprises
outputting an analysis result of a previous biological sample of a subject from whom the biological sample has been collected, and/or information about clinical information obtained by diagnosing the subject.

10. The analysis method of claim 8 or 9, further comprising outputting a message for suggesting re-analysis of the biological sample when a result outputted from the deep learning algorithm satisfies the second condition.

11. The analysis method of claim 10, further comprising

receiving an execution instruction for executing re-analysis of the biological sample when a result outputted by the deep learning algorithm satisfies the second condition, and
executing the re-analysis of the biological sample when the execution instruction is received.

12. The analysis method of claim 11, wherein the receiving of the execution instruction comprises receiving an execution instruction for analyzing an antigen or an antibody of a kind different from a kind of the antigen or the antibody contained in the biological sample.

13. The analysis method of claim 11 or 12, wherein the receiving of the execution instruction comprises receiving an execution instruction for analyzing an antigen or an antibody of a same kind as a kind of the antigen or the antibody contained in the biological sample by diluting the biological sample at a dilution rate higher than a dilution rate for the measurement sample.

14. An analyzer for analyzing an antigen or an antibody contained in a biological sample by using a measurement reagent containing an antibody or an antigen that causes antigen-antibody reaction with the antigen or the antibody in the biological sample, the analyzer comprising:

a sample preparation unit configured to prepare a measurement sample containing the biological sample and the measurement reagent;
a light applying unit configured to apply light to the measurement sample;
a detector configured to detect the light applied by the light applying unit, through the measurement sample, and output detection information corresponding to the detected light; and
a controller configured to

analyze the antigen or the antibody contained in the biological sample; and
detect occurrence of nonspecific reaction in the measurement sample, wherein the detecting of occurrence of the nonspecific reaction comprises the detection method according to any one of claims 1-3,

generating a data group about progress of antigen-antibody reaction between the antigen or the antibody contained in the biological sample and the antibody or the antigen contained in the measurement reagent based on the detection information,
inputting the data group to a deep learning algorithm, and
generating information about occurrence of nonspecific reaction based on a result outputted from the deep learning algorithm.

15. A detection program for detecting occurrence of nonspecific reaction, the detection program causing a computer to execute, when the computer is caused to execute the program the detection method according to any one of claims 1-3

**Patentansprüche**

1. Computer-implementiertes Detektionsverfahren zur Detektion des Auftretens einer unspezifischen Reaktion in der Analyse von einem Antigen oder einem Antikörper enthalten in einer biologischen Probe, unter Verwendung von einem Messreagens, das einen Antikörper oder ein Antigen enthält, das eine Antigen-Antikörper-Reaktion mit dem Antigen oder dem Antikörper in der biologischen Probe verursacht, das Detektionsverfahren umfassend:

Erzeugen einer Datengruppe über den Verlauf von der Antigen-Antikörper-Reaktion zwischen dem in der biologischen Probe enthaltenen Antigen oder Antikörper und dem im Messreagenz enthaltenen Antikörper oder Antigen;

Eingeben der Datengruppe in einen Deep-Learning-Algorithmus; und

Erzeugen von Informationen über das Auftreten einer unspezifischen Reaktion basierend auf einem Ergebnis, das von dem Deep-Learning-Algorithmus ausgegeben wird, wobei

das Erzeugen der Datengruppe das Empfangen von Detektionsinformation umfasst, die eine Intensität des delektierten Lichts angibt, das durch eine Messprobe detektiert wird, auf die Licht für eine vorbestimmte Zeitperiode angewendet wird, und Erzeugen der Datengruppe aus den Detektionsinformationen, die empfangen wurde, wobei jedes Datenelement erhalten in der Datengruppe, eine Intensität des detektierten Lichts ausdrückt, das zu jedem Messzeitpunkt in der vorbestimmten Zeitdauer detektiert wird.

2. Detektionsverfahren gemäß Anspruch 1, wobei jedes in der Datengruppe enthaltene Datenelement eine Intensität des durch die Messprobe durchgelassenen Lichts, eine Intensität des von der Messprobe absorbierten Lichts oder eine Intensität des von der Messprobe gestreuten Lichts angibt.

3. Das Detektionsverfahren gemäß Anspruch 1 oder 2, wobei die Detektionsinformation eine Intensität von dem detektierten Licht angibt, das durch die Messprobe detektiert wird, auf die eine Vielzahl von Lichtarten mit unterschiedlichen Wellenlängen sequentiell angewendet werden,

das Erzeugen der Datengruppe das Erzeugen einer Vielzahl von den Datengruppen jeweils entsprechend den Lichtern, basierend auf der Detektionsinformationen, umfasst, und

das Eingeben der Datengruppe in den Deep-Learning-Algorithmus das Eingeben in einer Vielzahl der Datengruppen in den Deep-Learning-Algorithmus umfasst.

4. Analyseverfahren zum Analysieren eines Antigens oder eines Antikörpers enthalten in biologische Probe unter Verwenden eines Messreagens, das einen Antikörper oder ein Antigen enthält, das eine Antigen-Antikörper-Reaktion mit dem Antigen oder dem Antikörper in der biologischen Probe verursacht, das Analyseverfahren umfassend:

Herstellen einer Messprobe enthaltend die biologische Probe und das Messreagenz;

Analysieren des in der biologischen Probe enthaltenen Antigens oder Antikörpers; und

Durchführen des Detektionsverfahrens gemäß Anspruch 1 zum Detektieren des Auftretens der unspezifischen Reaktion.

5. Analyseverfahren gemäß Anspruch 4, ferner umfassend: Anwenden von Licht auf die Messprobe für einen vorbestimmten Zeitperiode und Ausgeben von Detektionsinformation, die eine Intensität des detektierten Lichts angibt, das durch die Messprobe detektiert wird, wobei

das Analysieren des in der biologischen Probe enthaltenen Antigens oder Antikörpers das Analysieren des Antigens oder des Antikörpers basierend auf der Detektionsinformation umfasst.

6. Analyseverfahren gemäß Anspruch 5, wobei

das Erzeugen der Datengruppe umfasst Erzeugen der Datengruppe aus der Detektionsinformation, und

das Analysieren von dem in der biologischen Probe enthaltenen Antigen oder Antikörper umfasst Analysieren des Antigens oder des Antikörpers basierend auf der erzeugten Datengruppe generiert aus der Detektionsinformation.

7. Analyseverfahren gemäß irgendeinem der Ansprüche 4 bis 6, wobei, wenn ein Ergebnis von der Analyse des in der biologischen Probe enthaltenen Antigens oder Antikörpers eine erste Bedingung erfüllt, das Detektionsverfahren zum Detektieren des Auftretens der unspezifischen Reaktion durchgeführt wird.

8. Analyseverfahren gemäß irgendeinem der Ansprüche 4 bis 7, wobei, wenn ein von dem Deep-Learning-Algorithmus ausgegebenes Ergebnis eine zweite Bedingung erfüllt, Informationen über das Auftreten der unspezifischen Reaktion ausgegeben werden.

9. Analyseverfahren gemäß Anspruch 8, wobei das Ausgeben der Information über das Auftreten der unspezifischen Reaktion ferner umfasst

Ausgeben eines Analyseergebnisses einer früheren biologischen Probe von einem Subjekt, von dem die biologische Probe entnommen wurde, und/oder Informationen über klinische Informationen, die durch die Diagnose des Subjekts erhalten wurden.

10. Das Analyseverfahren gemäß Anspruch 8 oder 9, ferner umfassend Ausgeben einer Nachricht zum Vorschlagen einer Neuanalyse der biologischen Probe, wenn ein von dem Deep-Learning-Algorithmus ausgegebenes Ergebnis eine zweite Bedingung erfüllt.

11. Das Analyseverfahren gemäß Anspruch 10, ferner umfassend Empfangen einer Ausführungsanweisung zum Ausführen einer Neuanalyse von der biologischen Probe, wenn ein von dem Deep-Learning-Algorithmus ausgegebenes Ergebnis eine zweite Bedingung erfüllt, und
Ausführen der Neuanalyse der biologischen Probe, wenn die Ausführungsanweisung empfangen wird.

12. Analyseverfahren gemäß Anspruch 11, wobei das Empfangen der Ausführungsanweisung das Empfangen einer Ausführungsanweisung zur Analyse eines Antigens oder eines Antikörpers einer Art, unterschiedlich zu einer Art des in der biologischen Probe enthaltenen Antigens oder Antikörpers, umfasst.

13. Analyseverfahren gemäß Anspruch 11 oder 12, wobei das Empfangen der Ausführungsanweisung das Empfangen einer Ausführungsanweisung zum Analysieren eines Antigens oder eines Antikörpers derselben Art wie eine Art des in der biologischen Probe enthaltenen Antigens oder Antikörpers durch Verdünnen der biologischen Probe mit einer Verdünnungsrate, höher als eine Verdünnungsrate für die Messprobe, umfasst.

14. Analysegerät zum Analysieren eines Antigens oder eines Antikörpers enthalten in einer biologischen Probe, unter Verwenden eines Messreagens, das einen Antikörper oder ein Antigen enthält, das eine Antigen-Antikörper-Reaktion mit dem Antigen oder dem Antikörper in der biologischen Probe verursacht, das Analysegerät umfassend:

eine Probenvorbereitungseinheit, die so konfiguriert ist, dass sie eine Messprobe vorbereitet, die die biologische Probe und das Messreagenz enthält;
eine Beleuchtungseinheit, die so konfiguriert ist, dass sie Licht auf die Messprobe anwendeten;
einen Detektor, der so konfiguriert ist, dass er das von der Beleuchtungseinheit angewendete Licht erfasst, durch die Messprobe, und Ausgeben von Detektionsinformation, die dem erfassten Licht entspricht; und
einen Controller, der so konfiguriert ist, dass er das/den in der biologischen Probe enthaltene Antigen oder Antikörper analysiert; und
das Auftreten einer unspezifischen Reaktion in der Messprobe detektiert, wobei das Detektieren des Auftretens der unspezifischen Reaktion das Detektionsverfahren gemäß irgendeinem der Ansprüche 1-3 umfasst,
eine Datengruppe über den Verlauf der Antigen-Antikörper-Reaktion zwischen dem in der biologischen Probe enthaltenen Antigen oder Antikörper und dem in dem Messreagenz enthaltenen Antikörper oder Antigen basierend auf der Detektionsinformationen, erzeugt,
die Datengruppe in einen Deep-Learning-Algorithmus eingibt,
und Informationen über das Auftreten von unspezifischen Reaktionen basierend auf einem Ergebnis, das vom Deep-Learning-Algorithmus ausgegebenen wird, generiert.

15. Detektionsprogramm zum Detektieren des Auftretens einer unspezifischen Reaktion, das Detektionsprogramm veranlasst einen Computer, wenn der Computer veranlasst wird das Programm auszuführen, das Programm des Detektionsverfahrens gemäß irgendeinem der Ansprüche 1-3 auszuführen.

## Revendications

1. Procédé de détection mis en œuvre par ordinateur pour détecter une occurrence d'une réaction non spécifique lors de l'analyse d'un antigène ou d'un anticorps contenu dans un échantillon biologique avec utilisation d'un réactif de mesure contenant un anticorps ou un antigène qui provoque une réaction antigène-anticorps avec l'antigène ou l'anticorps dans l'échantillon biologique, le procédé de détection comprenant :

une génération d'un groupe de données concernant la progression d'une réaction antigène-anticorps entre l'antigène ou l'anticorps contenu dans l'échantillon biologique et l'anticorps ou l'antigène contenu dans le réactif de mesure ;
une entrée du groupe de données dans un algorithme d'apprentissage profond ; et une génération d'informations concernant une occurrence d'une réaction non spécifique, sur la base d'un résultat délivré en sortie par l'algorithme d'apprentissage profond, dans lequel
la génération du groupe de données comprend une réception d'informations de détection indiquant une intensité de lumière détectée qui est détectée à travers un échantillon de mesure auquel une lumière est appliquée pendant une période de temps prédéterminée, et une génération du groupe de données à partir des informations de détection ayant été reçues, dans lequel chaque élément de données inclus dans le groupe de données exprime une intensité de la lumière détectée qui est détectée à chaque moment de mesure dans la période de temps prédéterminée.

2. Procédé de détection selon la revendication 1, dans lequel chaque élément de données inclus dans le groupe de données indique une intensité de lumière transmise à travers l'échantillon de mesure, une intensité de lumière absorbée par l'échantillon de mesure, ou une intensité de lumière diffusée par l'échantillon de mesure.

3. Procédé de détection selon la revendication 1 ou la revendication 2, dans lequel

les informations de détection indiquent une intensité de la lumière détectée qui est détectée à travers l'échantillon de mesure auquel une pluralité de types de lumières ayant des longueurs d'onde différentes sont appliquées séquentiellement,
la génération du groupe de données comprend une génération d'une pluralité des groupes de données correspondant aux lumières, respectivement, sur la base des informations de détection, et
l'entrée du groupe de données dans l'algorithme d'apprentissage profond comprend une entrée d'une pluralité des groupes de données dans l'algorithme d'apprentissage profond.

4. Procédé d'analyse pour analyser un antigène ou un anticorps contenu dans un échantillon biologique en utilisant un réactif de mesure contenant un anticorps ou un antigène qui provoque une réaction antigène-anticorps avec l'antigène ou l'anticorps dans l'échantillon biologique, le procédé d'analyse comprenant :

une préparation d'un échantillon de mesure contenant l'échantillon biologique et le réactif de mesure ;
une analyse de l'antigène ou de l'anticorps contenu dans l'échantillon biologique ; et
une mise en œuvre du procédé de détection selon la revendication 1 pour détecter une occurrence de la réaction non spécifique.

5. Procédé d'analyse selon la revendication 4, comprenant en outre

une application d'une lumière à l'échantillon de mesure pendant une période de temps prédéterminée et une délivrance en sortie d'informations de détection indiquant une intensité de lumière détectée qui est détectée à travers l'échantillon de mesure, dans lequel
l'analyse de l'antigène ou de l'anticorps contenu dans l'échantillon biologique comprend une analyse de l'antigène ou de l'anticorps sur la base des informations de détection.

6. Procédé d'analyse selon la revendication 5,

dans lequel la génération du groupe de données comprend
une génération du groupe de données à partir des informations de détection, et
l'analyse de l'antigène ou de l'anticorps contenu dans l'échantillon biologique comprend
une analyse de l'antigène ou de l'anticorps sur la base du groupe de données généré à partir des informations de détection.

7. Procédé d'analyse selon l'une quelconque des revendications 4 à 6, dans lequel, lorsqu'un résultat de l'analyse de l'antigène ou de l'anticorps contenu dans l'échantillon biologique satisfait à une première condition, le procédé de détection pour détecter une occurrence de la réaction non spécifique est effectué.

8. Procédé d'analyse selon l'une quelconque des revendications 4 à 7, dans lequel, lorsqu'un résultat délivré en sortie par l'algorithme d'apprentissage profond satisfait à une seconde condition, des informations concernant une occurrence de la réaction non spécifique sont délivrées en sortie.

9. Procédé d'analyse selon la revendication 8, dans lequel
la délivrance en sortie des informations concernant une occurrence de la réaction non spécifique comprend en outre une délivrance en sortie d'un résultat d'analyse d'un échantillon biologique précédent d'un sujet duquel l'échantillon biologique a été prélevé, et/ou d'informations concernant des informations cliniques obtenues en diagnostiquant le sujet.

10. Procédé d'analyse selon la revendication 8 ou la revendication 9, comprenant en outre une délivrance en sortie d'un message pour suggérer une réanalyse de l'échantillon biologique lorsqu'un résultat délivré en sortie par l'algorithme d'apprentissage profond satisfait à la seconde condition.

11. Procédé d'analyse selon la revendication 10, comprenant en outre

une réception d'une instruction d'exécution pour exécuter une réanalyse de l'échantillon biologique lorsqu'un résultat délivré en sortie par l'algorithme d'apprentissage profond satisfait à la seconde condition, et
une exécution de la réanalyse de l'échantillon biologique lors de la réception de l'instruction d'exécution.

12. Procédé d'analyse selon la revendication 11, dans lequel la réception de l'instruction d'exécution comprend une réception d'une instruction d'exécution pour analyser un antigène ou un anticorps d'un type différent d'un type de l'antigène ou de l'anticorps contenu dans l'échantillon biologique.

13. Procédé d'analyse selon la revendication 11 ou la revendication 12, dans lequel la réception de l'instruction d'exécution comprend une réception d'une instruction d'exécution pour analyser un antigène ou un anticorps d'un même type qu'un type de l'antigène ou de l'anticorps contenu dans l'échantillon biologique en diluant l'échantillon biologique à un taux de dilution supérieur à un taux de dilution pour l'échantillon de mesure.

14. Analyseur pour analyser un antigène ou un anticorps contenu dans un échantillon biologique en utilisant un réactif de mesure contenant un anticorps ou un antigène qui provoque une réaction antigène-anticorps avec l'antigène ou l'anticorps dans l'échantillon biologique, l'analyseur comprenant :

une unité de préparation d'échantillon configurée pour préparer un échantillon de mesure contenant l'échantillon biologique et le réactif de mesure ;
une unité d'application de lumière configurée pour appliquer une lumière à l'échantillon de mesure ;
un détecteur configuré pour détecter la lumière appliquée par l'unité d'application de lumière, à travers l'échantillon de mesure, et délivrer en sortie des informations de détection correspondant à la lumière détectée ; et
un dispositif de commande configuré pour

analyser l'antigène ou l'anticorps contenu dans l'échantillon biologique ; et
détecter une occurrence d'une réaction non spécifique dans l'échantillon de mesure, dans lequel la détection d'une occurrence de la réaction non spécifique comprend le procédé de détection selon l'une quelconque des revendications 1 à 3,
une génération d'un groupe de données concernant la progression d'une réaction antigène-anticorps entre l'antigène ou l'anticorps contenu dans l'échantillon biologique et l'anticorps ou l'antigène contenu dans le réactif de mesure sur la base des informations de détection,
une entrée du groupe de données dans un algorithme d'apprentissage profond, et
une génération d'informations concernant une occurrence d'une réaction non spécifique sur la base d'un résultat délivré en sortie par l'algorithme d'apprentissage profond.

15. Programme de détection pour détecter une occurrence d'une réaction non spécifique, le programme de détection amenant un ordinateur à exécuter, lorsque l'ordinateur est amené à exécuter le programme, le procédé de détection selon l'une quelconque des revendications 1 à 3.

FIG. 1

# FIG. 2

FIG. 3

STORAGE UNIT — 202

- MEASUREMENT PROGRAM — 202a
- TEST SUBSTANCE ANALYSIS/NONSPECIFIC REACTION DETECTION PROGRAM — 202b
- DEEP LEARNING ALGORITHM DATABASE — DB1
- CALIBRATION CURVE/THRESHOLD VALUE DATABASE — DB2
- RE-ANALYSIS ITEM DATABASE — DB3
- RE-ANALYSIS RESULT DATABASE — DB4

FIG. 4

FIG. 5A

FIG. 5B

FIG. 6

ANALYSIS PROCESS

FIG. 7

MEASUREMENT PROCESS

```
        ( START )
            |
            v
+---------------------------+
| OBTAIN MEASUREMENT ITEM   |   S11
|       INFORMATION         |
+---------------------------+
            |
            v
+---------------------------+
| DISPENSE BIOLOGICAL SAMPLE|   S12
+---------------------------+
            |
            v
+---------------------------+
| DISPENSE MEASUREMENT REAGENT| S13
+---------------------------+
            |
            v
+---------------------------+
|       MEASUREMENT         |   S14
+---------------------------+
            |
            v
        ( RETURN )
```

# FIG. 8

TEST SUBSTANCE ANALYSIS/NONSPECIFIC
REACTION DETECTION PROCESS

START

RECEIVE DETECTION INFORMATION AND GENERATE DATA GROUP
ABOUT PROGRESS OF ANTIGEN-ANTIBODY REACTION | S21

ANALYZE TEST SUBSTANCE
(OBTAIN CONCENTRATION OF TEST SUBSTANCE) | S22

S23
DOES CONCENTRATION EXCEED
THRESHOLD VALUE? — No

Yes

INPUT DATA GROUP TO DEEP LEARNING ALGORITHM AND
CALCULATE PROBABILITY | S24

S25
DOES PROBABILITY EXCEED
THRESHOLD VALUE? — No

Yes

GENERATE INFORMATION ABOUT OCCURRENCE OF
NONSPECIFIC REACTION, AND OUTPUT CONCENTRATION
OF TEST SUBSTANCE AND INFORMATION ABOUT
OCCURRENCE OF NONSPECIFIC REACTION | S26

OUTPUT CONCENTRATION | S27

RETURN

FIG. 9A

DATA GROUP ABOUT PROGRESS OF
ANTIGEN-ANTIBODY REACTION

| | | | | | | | | | | |
|---|---|---|---|---|---|---|---|---|---|---|
| FIRST WAVELENGTH | 749 | 752 | 750 | 757 | 763 | ... | ... | ... | 1148 | 1150 | D11 |
| SECOND WAVELENGTH | 760 | 761 | 760 | 770 | 773 | ... | ... | ... | 1149 | 1152 | D12 |
| THIRD WAVELENGTH | 769 | 752 | 750 | 759 | 764 | ... | ... | ... | 1145 | 1160 | D13 |
| FOURTH WAVELENGTH | 770 | 777 | 780 | 789 | 790 | ... | ... | ... | 1148 | 1167 | D14 |
| FIFTH WAVELENGTH | 755 | 752 | 757 | 759 | 761 | ... | ... | ... | 1138 | 1140 | D15 |

FIG. 9B

FIG. 10

MB1

MB11

MEASUREMENT DATE: 2020/7/XX

MEASUREMENT TIME: HH/MM/SS

BIOLOGICAL SAMPLE ID: ▲▲▲

<MEASUREMENT RESULT>

DD C.: 30.5 μg/mL
FDP C.: 10.1 μg/mL

MB12

<MESSAGE>

SUSPICION OF NONSPECIFIC REACTION FOR DD C: +++ (98.4%)

FIG. 11

FIRST TRAINING DATA

SECOND TRAINING DATA  OCCURRENCE OF NONSPECIFIC REACTION: PRESENT/ABSENT

FIG. 12

DATA GROUP ABOUT PROGRESS OF
ANTIGEN-ANTIBODY REACTION

d11 d12 d13 · · ·

| | | | | | | | | |
|---|---|---|---|---|---|---|---|---|
| FIRST WAVELENGTH | 749 | 752 | 750 | 757 | 763 | ... | ... | ... | 1148 | 1150 | D11 / D12 |
| SECOND WAVELENGTH | 760 | 761 | 760 | 770 | 773 | ... | ... | ... | 1149 | 1152 | D13 |
| THIRD WAVELENGTH | 769 | 752 | 750 | 759 | 764 | ... | ... | ... | 1145 | 1160 | |
| FOURTH WAVELENGTH | 770 | 777 | 780 | 789 | 790 | ... | ... | ... | 1148 | 1167 | D14 |
| FIFTH WAVELENGTH | 755 | 752 | 757 | 759 | 761 | ... | ... | ... | 1138 | 1140 | D15 |

60

60b

60a    60c

PROBABILITY OF OCCURRENCE OF NONSPECIFIC REACTION: 98.4%

FIG. 13

MB1'

MB11'

MEASUREMENT DATE: 2020/7/XX

MEASUREMENT TIME: HH/MM/SS

BIOLOGICAL SAMPLE ID: ▲▲▲


<MEASUREMENT RESULT>

DD C.: 30.5μg/mL (PREVIOUS VALUE: 10.1μg/mL)

FDP C.: 10.1μg/mL

MB12'

<MESSAGE>

SUSPICION OF NONSPECIFIC REACTION: +++ (98.4%)
IN THE CASE OF DISCREPANCY BETWEEN CLINICAL SYMPTOM AND ANOTHER TEST RESULT, MEASUREMENT RESULT IS SUSPECTED TO INDICATE FALSE HIGH VALUE DUE TO NONSPECIFIC REACTION.

MB13

<CLINICAL FINDING>

THROMBUS TENDS TO BE FOUND. UNDER ANTICOAGULANT THERAPY.

FIG. 14

RE-ANALYSIS
PROCESS

( FROM STEP S26 )

↓

| OUTPUT DIALOG BOX | S28

↓

S29

< SELECT SELECTION REGION MB334? > ──No──→

Yes ↓

S30

< SELECT SELECTION REGION MB331? > ──No──→

Yes ↓

( TO STEP S12 )

S31

< SELECT SELECTION REGION MB332? > ──No──→

Yes ↓

( TO STEP S11 )

S32

< SELECT SELECTION REGION MB333? > ──No──→

Yes ↓

| DISPENSE AND DILUTE BIOLOGICAL SAMPLE | S12'

↓

( TO STEP S13 )

( FROM STEP S27 )

( RETURN )

EP 4 109 098 B1

FIG. 15

MB3

CONFIRMATION OF RE-ANALYSIS    MB31

NONSPECIFIC REACTION IS LIKELY TO HAVE OCCURRED FOR D-DIMER.
PERFORM RE-ANALYSIS?

MB33

| DD RE-ANALYSIS | FDP ANALYSIS | DILUTION AND ANALYSIS | IGNORE |

MB331    MB332    MB333    MB334

FIG. 16

DB3

| | | | | |
|---|---|---|---|---|
| MEASUREMENT ITEM FOR INITIAL MEASUREMENT | D-DIMER | FDP | FMC | VWF:Ag |
| MEASUREMENT ITEM FOR RE-ANALYSIS | FDP | D-DIMER | SF、 TAT、 F1+2 | VWF:RCo、 VWF:GPIbR、 VWF:GPIbM、 VWF:Ab |

FIG. 17

MB1″

MB11″

MEASUREMENT DATE: 2020/7/XX

MEASUREMENT TIME: HH/MM/SS

BIOLOGICAL SAMPLE ID: ▲▲▲

<MEASUREMENT RESULT>

DD C.: 0.5 μg/mL   (RESULT OF RE-ANALYSIS)

DD C.: 30.5 μg/mL $\left(\begin{array}{c}\text{RESULT OF INITIAL}\\\text{ANALYSIS}\end{array}\right)$ (REFERENCE VALUE)

MB12″

<MESSAGE>

SINCE NONSPECIFIC REACTION IS SUSPECTED TO HAVE OCCURRED IN
INITIAL ANALYSIS, ADDITIONAL ANALYSIS HAS BEEN PERFORMED.

FIG. 18

DB4

| IDENTIFICATION INFORMATION (ID) OF BIOLOGICAL SAMPLE | DATA GROUP | PRESENCE OR ABSENCE OF OCCURRENCE OF NONSPECIFIC AGGLUTINATION REACTION |
|---|---|---|
| 0 0 0 0 0 1 | FIRST WAVELENGTH: 759 762 760 767 773 ... ... ... 1148 1150<br>SECOND WAVELENGTH: 730 761 750 773 774 ... ... ... 1139 1142<br>THIRD WAVELENGTH: 759 752 751 761 764 ... ... ... 1115 1161<br>FOURTH WAVELENGTH: 770 787 789 790 790 ... ... ... 1138 1157<br>FIFTH WAVELENGTH: 745 742 757 769 765 ... ... ... 1134 1150 | PRESENT |
| 0 0 0 0 0 4 | FIRST WAVELENGTH: 749 752 750 757 763 ... ... ... 1148 1150<br>SECOND WAVELENGTH: 760 761 760 770 773 ... ... ... 1149 1152<br>THIRD WAVELENGTH: 769 752 750 759 764 ... ... ... 1145 1160<br>FOURTH WAVELENGTH: 770 777 780 789 790 ... ... ... 1148 1167<br>FIFTH WAVELENGTH: 755 752 757 759 761 ... ... ... 1138 1140 | PRESENT |
| 0 0 0 0 1 0 | · · · · ·<br>· · · · ·<br>· · · · ·<br>· · · · · | ABSENT |

FIG. 19A

FIG. 19B

FIG. 20

START

GENERATE S VALUE DISTRIBUTION OF BIOLOGICAL SAMPLE GROUP FOR WHICH NONSPECIFIC REACTION DOES NOT OCCUR    S81

CALCULATE S VALUE OF BIOLOGICAL SAMPLE TO BE ANALYZED    S82

CALCULATE PROBABILITY OF OCCURRENCE OF NONSPECIFIC REACTION    S83

RETURN

FIG. 21

| PARAMETER S[-] | 2.25 | 3.9 |
|---|---|---|
| NONSPECIFIC REACTION P[%] | 0 | 100 |

FIG. 22

FIG. 23

SENSITIVITY 62%

SPECIFICITY 96%

AUC=0.899

**EP 4 109 098 B1**

**Patent documents cited in the description**

- EP 0566205 A

- US 10048249 B

**Non-patent literature cited in the description**

- **FRUTIGER, ANDREAS et al.** Nonspecific binding-fundamental concepts and consequences for bio-sensing applications. *Chemical reviews*, 2021, vol. 121 (13), 8095-8160